# EUROPÄISCHE PATENTSCHRIFT

(11) **EP 1 274 709 B1**
(45) Veröffentlichungstag und Bekanntmachung des Hinweises auf die Patenterteilung: **22.09.2004**
(21) Anmeldenummer: 01931560.5
(22) Anmeldetag: 03.04.2001
(51) Int. Cl.: C07D 471/04, C07D 487/04, C07D 513/04, A61K 31/435, A61K 31/495, A61K 31/425, A61K 31/415

(54) **SALZE VON BICYCLISCHEN, N-ACYLIERTEN IMIDAZO-3-AMINEN UND IMIDAZO-5-AMINEN**
SALTS OF BICYCLIC, N-ACYLATED IMIDAZO-3-AMINES AND IMIDAZO-5-AMINES
SELS D'IMIDAZO-3-AMINES ET D'IMIDAZO-5-AMINES N-ACYLEES, BICYCLIQUES

(30) Priorität: 20.04.2000 DE 10019714
(43) Veröffentlichungstag der Anmeldung: 15.01.2003
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE)
(86) Internationale Anmeldenummer: PCT/EP2001/003772
(87) Internationale Veröffentlichungsnummer: WO 2001/081344

(56) Entgegenhaltungen:
- EP-A- 0 353 047
- GB-A- 2 039 882
- US-A- 3 894 022
- US-A- 4 183 932
- US-A- 4 650 796
- US-A- 5 068 244
- US-A- 5 536 853

## Beschreibung

Die vorliegende Erfindung betrifft Salze von bicyclischen, N-acylierten Imidazo-3-aminen und Imidazo-5-aminen, ein Verfahren zu deren Herstellung, deren Verwendung zur Herstellung von Arzneimitteln und Arzneimittel enthaltend diese Verbindungen.

Für einzelne Verbindungen aus der Klasse der den Grundkörper der erfindungsgemäßen Verbindungen bildenden, nicht acylierten, bicyclischen Imidazo-3-amine und Imidazo-5-amine sind interessante pharmakologische Eigenschaften bekannt. So werden bestimmte Imidazo[1,2-a]pyridine als den Blutdruck senkende Wirkstoffe (GB-B-1,135,893), als Anthelminthika und Antimykotika (J. Med. Chem. 1972, 15, 982-985)und als antisekretorische Wirkstoffe zur Behandlung von entzündlichen Erkrankungen (EP-A-0 068 378) beschrieben. Eine Wirkung einzelner Imidazopyridine gegen entzündliche Erkrankungen insbesondere des Magens beschreiben auch EP-A-0 266 890 und J. Med. Chem. 1987, 30, 2031-2046. Weitere, für einzelne Vertreter aus der Klasse der nicht acylierten Imidazo-3-amine und Imidazo-5-amine beschriebene pharmakologische Wirkungen sind antibakterielle Eigenschaften (Chem. Pharm. Bull. 1992, 40, 1170), antivirale Eigenschaften (J. Med. Chem. 1998, 41, 5108-5112) sowie die Wirkung als Benzodiazepin-Rezeptor Antagonist (J. Heterocyclic Chem. 1998,35, 1205-1217).

EP-A-0 353 047 beschreibt die Verwendung von Imidazopyrazolen als analgetische und antiinflamatorische Wirkstoffe. Diese Verbindungen weisen jedoch am Imidazol-Ring keine exocyclische Aminogruppe auf.

US-A-3 894 022 beschreibt Imidazolin-Derivate, die am Imidazolinring alkylsubstituiert sein können und ebenfalls analgetische und antiinflamatorische Wirkungen aufweisen.

Aus der US-A- 4 650 796 sind Imidazo[1,2-a]pyridine mit u. a. analgetischer Wirkung bekannt, die in 3-Position eine über eine CH₂-Gruppe gebundene Säuramidgruppe aufweisen.

Weiterhin beschreibt die GB-A-2 039 882 analgetisch wirksame, in 3-Position mit einem aromatischen Ring substituierte Imidazo-dihydrothiazol-Derivate, also Verbindungen, bei denen der mit dem Imidazolring verbundene, ein Schwefelatom enthaltende Fünfring keine Doppelbindung enthält.

Außerdem fallen unter die in US-A-4 183 932 offenbarte allgemeine Formel auch einige bicyclische Imidazo-Verbindungen, die jedoch weder am Stickstoff in 1-Position des Imidazolringes noch in dessen 3-Position einen Substituenten aufweisen.

Vermehrtes Interesse hat die Klasse der nicht acylierten, bicyclischen Imidazo-3-amine und Imidazo-5-amine dadurch gefunden, daß zu ihrer Synthese Mehrkomponentenreaktionen entwickelt wurden, die sich für eine automatisierte, kombinatorische Chemie eignen. Während in üblichen Reaktionssequenzen das isolierte Zwischenprodukt im nächsten Schritt weiterreagiert, finden bei Mehrkomponentenreaktionen Gleichgewichtsreaktionen zwischen den Edukten und diversen Zwischenprodukten statt, wobei ein stabiles Produkt entsteht. Effizient ist die Mehrkomponentenreaktion vor allem, wenn das gewünschte Produkt im Gleichgewichtszustand stark überwiegt, oder sogar durch eine irreversible Reaktionsführung aus dem Gleichgewicht entfernt wird. Idealerweise sollten darüberhinaus in einer für die kombinatorische Chemie verwendbaren Mehrkomponentenreaktion möglichst viele, variable und einfach erhältliche Edukte verwendbar sein.

So beschreiben C. Blackburn et al. in Tetrahedron Lett. 1998, 39, 3635-3638 eine Dreikomponenten-Kondensation zur Parallelsynthese von bicyclischen Imidazo-3-aminen und Imidazo-5-aminen. Ähnlich der dieser Reaktion ist die in von K. Groebke et al. in Synlett 1998, 661-663 publizierte Synthese. Eine Mehrkomponentenreaktion für die kombinatorische Synthese von bicyclischen Imidazo-3-aminen, mit der auch vereinzelte Imidazo-5-amine herstellt wurden, beschreiben auch H. Bienayme und K. Bouzid in Angew. Chem. 1998, 110 (16), 2349-2352.

N-acylierte, bicyclische Imidazo-3-amine waren bisher nur in soweit bekannt, daß Chayer et al. in Tetrahedron Lett. 1998,39, 9685-9688 Salze N-acylierter, aber in 3-Position unsubstituierter Imidazo-[1,2-a]-pyridine als Zwischenstufe bei der Herstellung der entsprechenden, in 3-Position C-acylierten Verbindungen beschreiben.

Darüberhinaus ist für einzelne, durch Festphasensynthese hergestellte bicyclische Imidazo-3-amine lediglich die N-Acylierung am Amino-Stickstoff beschrieben (Blackburn in Tetrahedron Lett. 1998, 39, 5469-5472).

Eine Aufgabe der vorliegenden Erfindung war es daher, erstmals Salze von bicyclischen, am Imidazol-Stickstoff N-acylierten Imidazo-3-aminen und Imidazo-5-aminen bereitzustellen.

Gelöst wurde diese Aufgabe erfindungsgemäß durch die Bereitstellung der Salze von bicyclischen, N-acylierten Imidazo-3-aminen und Imidazo-5-aminen der allgemeinen Formel I, 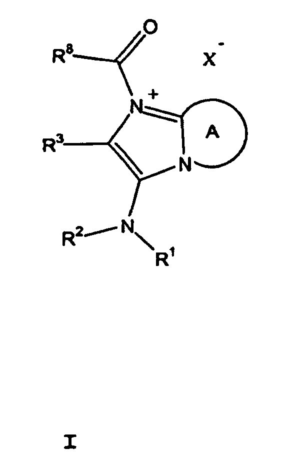 worin
R¹ *tert*-Butyl, 1,1,3,3-Tetramethylbutyl, (CH₂)₆CN, Cyclohexyl, CH₂CO(OMethyl), 2,6-Dimethylphenyl, *tert*-Butyl oder CH₂CH₂R (R = 4-Morpholino) bedeutet,
R² Wasserstoff, COR^{b}, wobei R^{b} für unverzweigtes oder verzweigtes C₁-C₈-Alkyl oder C₃-C₈-Cycloalkyl steht, bedeutet,
R³ ausgewählt ist aus der Gruppe 4-Acetamidophenyl, 2-Bromphenyl, 3-Bromphenyl, 4-Bromphenyl, 4-Brom-2-fluorphenyl, 5-Brom-2-fluorphenyl, 3-Brom-4-fluorphenyl, 4-tert. Butylphenyl, 2-Chlor-4-fluorphenyl, 2-Chlor-6-fluorphenyl, 2-Chlorphenyl, 3-Chlorphenyl, 4-Chlorphenyl, 4-Cyanophenyl, 2,3-Dichlorphenyl, 2,4-Dichlorphenyl, 3,4-Dichlorphenyl, 2,3-Dimethoxyphenyl, 3,4-Dimethoxyphenyl, 2,4-Dimethylphenyl, 2,5-Dimethylphenyl, 2-Fluor-phenyl, 3-Fluorphenyl, 4-Fluorphenyl, 4-Hexylphenyl, 3-Hydroxyphenyl, 2-Methoxyphenyl, 2-Methylphenyl, 3-Methylphenyl, 4-Methylphenyl, 4-Nitrophenyl, 3-Phenoxyphenyl, 4-(1-Pyrrolidino)phenyl, 2-(Trifluormethyl)phenyl, 3-(Trifluormethyl)phenyl, 4-(Trifluormethyl)phenyl, 3,4,5-Trimethoxyphenyl, 3-(4-Chlorphenoxy)phenyl, 4-Acetoxy-3-methoxyphenyl, 4-Dimethylaminonaphthyl, 2-Ethoxynaphthyl, 4-Methoxynaphthyl, 2-(1-(Phenylsulfonyl)-pyrrol), 2-(N-Methylpyrrol), 2-(N-(3,5-Dichlorphenyl)pyrrol), 2-(1-(4-Chlorphenyl)pyrrol), 2-(5-Acetoxymethylfurfuryl), 2-(5-Methylfurfuryl), 2-(5-Nitrofurfuryl), 2-[5-(3-Nitrophenyl)furfuryl], 2-[5-(2-Nitrophenyl)furfuryl], 2-(5-Bromfurfuryl), 2-[5-(4-Chlorphenyl)furfuryl], 2-(4,5-Dimethylfurfuryl), 2-[5-(2-Chlorphenyl]furfuryl], 2-(5-Ethylfurfuryl), 2-[5-(1,3-Dioxalanfurfuryl], 2-(5-Chlorthiophenyl), 2-(5-Methylthiophenyl), 2-(5-Ethylthiophenyl), 2-(3-Methylthiophenyl), 2-(4-Bromthiophenyl), 2-(5-Nitrothiophenyl), 5-(2-Carbonsäurethiophenyl), 2-[4-(Phenylethyl)-thiophenyl], 2-[5-(Methylthio)thiophenyl], 2-(3-Bromthiophenyl), 2-(3-Phenoxythiophenyl) oder 2-(5-Bromthiophenyl) oder insbesondere Methyl, Cyclohexyl, Phenyl, Furan-2-yl, 2-Pyridyl oder 2-Thiophenyl.
A ein dreigliedriges Ringfragment ausgewählt aus einer der Formeln 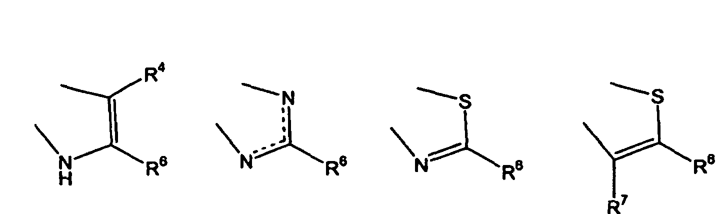 oder ein viergliedriges Ringfragment ausgewählt aus einer der Formeln 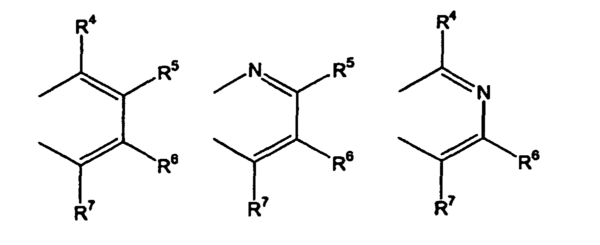 bedeutet, in dem jeweils R⁴, R⁵, R⁶ und R⁷ unabhängig voneinander Wasserstoff, unverzweigtes oder verzweigtes C₁-C₈-Alkyl, Fluor, Chlor, Brom, CF₃, CN, NO₂, NHR^{f}, wobei R^{f} für Wasserstoff, unverzweigtes oder verzweigtes C₁-C₈-Alkyl, oder COR''' (R''' = unverzweigtes oder verzweigtes C₁-C₄-Alkyl oder Phenyl) steht, SR^{g}, wobei R^{g} für Wasserstoff, unverzweigtes oder verzweigtes C₁-C₄-Alkyl, Phenyl, Pyridin, Benzyl oder substituiertes steht, OR^{h}, wobei R^{h} für Wasserstoff, unverzweigtes oder verzweigtes C₁-C₈-Alkyl, COR''' (R''' = unverzweigtes oder verzweigtes C₁-C₄-Alkyl oder Phenyl) oder CO(OR') (R' = unverzweigtes oder verzweigtes C₁-C₈-Alkyl) steht, CO(ORⁱ) oder CH₂CO(ORⁱ), wobei Rⁱ jeweils für verzweigtes oder unverzweigtes C₁-C₈-Alkyl oder substituiertes steht, bedeutet oder R⁶ und R⁷ gemeinsam ein Ringfragment -CRⁱ=CR^{j}-CH=CH- bedeuten, wobei Rⁱ und R^{j} Wasserstoff bedeuten oder einer der beiden Reste Rⁱ oder R^{j} von Wasserstoff verschieden ist und F, Cl, Br, I oder unverzweigtes oder verzweigtes C₁-C₈-Alkyl bedeutet, während R⁴ und R⁵ unabhängig davon die zuvor angegebene Bedeutung haben,
R⁸ verzweigtes oder unverzweigtes C₁-C₈-Alkyl, Aryl, Heteroaryl, C₃-C₈-Cycloalkyl oder einen über eine C₁-C₃-Alkylen-Gruppe gebundenen Aryl, Heteroaryl oder C₃-C₈-Cycloalkylrest bedeutet,
und
X das Anion einer anorganischen oder organischen Säure bedeutet.

Die Strichelung in der Formel 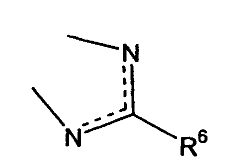 soll dabei bedeuten, daß sich zwischen einem der Stickstoff-Atome und dem mit R⁶ verbundenen C-Atom eine Doppelbindung befindet, während in dem anderen Stickstoff-Atom die freie Bindungsstelle durch ein Wasserstoffatom abgesättigt ist.

Erfindungsgemäß bevorzugt sind dabei Salze von bicyclischen, N-acylierten Imidazo-3-aminen und Imidazo-5-aminen der Formel I, bei denen R² Wasserstoff bedeutet.
Bevorzugt sind außerdem Salze von bicyclischen, N-acylierten Imidazo-3-aminen und Imidazo-5-aminen, bei denen A ein dreigliedriges Ringfragment der Formel 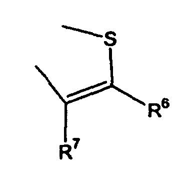 oder ein viergliedriges Ringfragment ausgewählt aus einer der Formeln 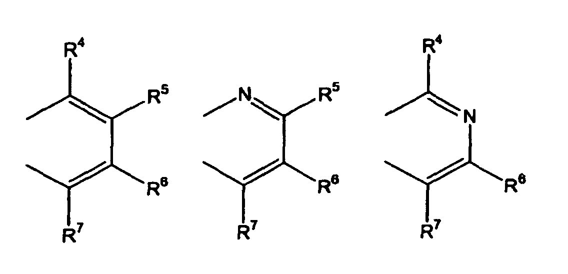 bedeutet.

Darüberhinaus sind in den erfindungsgemäßen Salzen von bicyclischen, N-acylierten Imidazo-3-aminen und Imidazo-5-aminen der Formel I die Reste R⁴, R⁵ R⁶ und R⁷ unabhängig voneinander bevorzugt ausgewählt sind aus der Gruppe Wasserstoff, Methyl, Ethyl, Isopropyl, n-Propyl, n-Butyl, Fluor, Brom, CF₃, CN, NO₂, NHR^{f}, wobei R^{f} für Wasserstoff, Methyl, Ethyl, n-Propyl, n-Butyl, COMethyl oder COPhenyl steht, SR^{g}, wobei R^{g} für Wasserstoff, Methyl, Ethyl, n-Propyl, n-Butyl oder Phenyl steht, OR^{h}, wobei R^{h} für Wasserstoff, Methyl, Ethyl, n-Propyl, n-Butyl, Phenyl, C(O)Methyl, C(O)Phenyl CO(OMethyl) oder CO(OEthyl) steht, CO(ORⁱ) oder CH₂CO(ORⁱ), wobei Rⁱ jeweils für Methyl, Ethyl, n-Propyl, n-Butyl oder Phenyl steht, wobei für R⁴, R⁵ R⁶ und R⁷ Wasserstoff, Methyl, Chlor, NH₂, und NO₂ besonders bevorzugt sind.

R⁸ bedeutet in den Salzen von bicyclischen, N-acylierten Imidazo-3-aminen und Imidazo-5-aminen der Formel I erfindungsgemäß vorzugsweise Methyl, Ethyl, n-Propyl, n-Butyl, n-Pentyl, n-Hexyl, unsubstituiertes, in 4-Position monosubstituiertes oder in 2- und 6-Position disubstituiertes Phenyl, Cyclohexyl oder 2-Naphtyl, wobei Methyl, n-Hexyl, 2,6-Dichlorphenyl, 4-Methoxyphenyl, Cyclohexyl oder 2-Naphtyl besonders bevorzugt sind.

Bei dem Anion X einer anorganischen oder organischen Säure handelt es sich erfindungsgemäß vorzugsweise um das Anion der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure oder Asparaginsäure oder insbesondere der Salzsäure.

Da die Salze der Formel I noch mindestens ein basisches Stickstoff-Atom aufweisen, lassen sie sich mit Säuren, vorzugsweise physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in bekannter Weise in Additionsprodukte überführen. Zur Herstellung des HCl-Additionsprodukts eignet sich darüber hinaus Trimethylchlorsilan in wäßriger Lösung. Diese Additionsprodukte sind gleichfalls Gegenstand der vorliegenden Erfindung.

Ganz besonders bevorzugt sind die erfindungsgemäßen Salze von bicyclischen, N-acylierten Imidazo-3-aminen und Imidazo-5-aminen und deren Additionsprodukte mit Säuren ausgewählt aus der Gruppe
1-Acetyl-3-*tert*-butylamino-2-phenyl-imidazo[1,2-a]-pyridin-1-iumchlorid
1-Acetyl-3-*tert*-butylamino-2-phenyl-imidazo[1,2-a]-pyrazin-1-iumchlorid
1-Acetyl-3-*tert*-butylamino-2-phenyl-imidazo[1,2-a]-pyrimidin-1-iumchlorid
7-Acetyl-5-*tert*-butylamino-6-phenyl-imidazo[2,1-b]-thiazol-7-iumchlorid
3-Acetyl-1-cyclohexylamino-2-phenyl-imidazo[1,2-a]-chinolin-3-iumchlorid
1-Acetyl-3-cyclohexylamino-2-phenyl-imidazo[1,2-a]-pyrimidin-1-iumchlorid
1-Acetyl-8-benzyloxy-3-cyclohexylamino-2-methylimidazo[1,2-a]pyridin-1-iumchlorid
1-Acetyl-8-benzyloxy-3-*tert*-butylamino-2-methylimidazo[1,2-a]pyridin-1-iumchlorid
1-Acetyl-8-benzyloxy-2-methyl-3-(1,1,3,3-tetramethylbutylamino)imidazo[1,2-a]pyridin-1-iumchlorid
3-(*tert*-Butyl-cyclohexancarbonyl-amino)-1-cyclohexancarbonyl-2-phenyl-imidazo[1,2-a]pyridin-1-iumchlorid
3-(*tert*-Butyl-heptanoyl-amino)-1-heptanoyl-2-phenylimidazo[1,2-a]pyridin-1-iumchlorid
1-Acetyl-3-cyclohexylamino-2-furan-2-yl-imidazo[1,2-a]-pyridin-1-iumchlorid
1-Acetyl-3-cyclohexylamino-2-furan-2-yl-imidazo[1,2-a]-pyrimidin-1-iumchlorid
1-Acetyl-5-amino-7-chlor-3-cyclohexylamino-2-furan-2-ylimidazo[1,2-a]pyrimidin-1-iumchlorid
1-Acetyl-3-cyclohexylamino-2-pyridin-2-yl-imidazo-[1,2-a]pyridin-1-iumchlorid
1-Acetyl-3-cyclohexylamino-6-nitro-2-pyridin-2-ylimidazo[1,2-a]pyridin-1-iumchlorid
1-Acetyl-3-cyclohexylamino-5-methyl-2-pyridin-2-ylimidazo[1,2-a]pyridin-1-iumchlorid
1-Acetyl-3-cyclohexylamino-2-pyridin-2-yl-imidazo[1,2-a]pyrazin-1-iumchlorid
1-Acetyl-3-cyclohexylamino-2-pyridin-2-yl-imidazo-[1,2-a]pyrimidin-1-iumchlorid
1-Acetyl-5-amino-7-chlor-3-cyclohexylamino-2-pyridin-2-ylimidazo[1,2-a]pyrimidin-1-iumchlorid
1-Acetyl-3-cyclohexylamino-5,7-dimethyl-2-pyridin-2-ylimidazo[1,2-a]pyrimidin-1-iumchlorid
7-Acetyl-5-cyclohexylamino-6-pyridin-2-yl-imidazo-[2,1-b]thiazol-7-iumchlorid
1-Acetyl-2-cyclohexyl-3-cyclohexylamino-imidazo[1,2-a]-pyridin-1-iumchlorid
1-Acetyl-3-cyclohexylamino-2-methyl-imidazo[1,2-a]-pyrimidin-1-iumchlorid
1-Acetyl-5-amino-7-chlor-3-cyclohexylamino-2-methylimidazo[1,2-a]pyrimidin-1-iumchlorid
1-Acetyl-5-amino-7-chlor-3-cyclohexylamino-2-thiophen-2-yl-imidazo[1,2-a]pyrimidin-1-iumchlorid
1-Acetyl-5-amino-7-chlor-2-furan-2-yl-3-(methoxycarbonylmethylamino)imidazo[1,2-a]pyrimidin-1-iumchlorid
1-Acetyl-2-cyclohexyl-3-(methoxycarbonylmethylamino)-imidazo[1,2-a]pyridin-1-iumchlorid
1-Acetyl-2-cyclohexyl-3-(methoxycarbonylmethylamino)-imidazo[1,2-a]pyrimidin-1-iumchlorid
1-Acetyl-3-(2,6-dimethyl-phenylamino)-2-methyl-6-nitroimidazo[1,2-a]pyridin-1-iumchlorid
1-Acetyl-5-amino-7-chlor-3-(2,6-dimethyl-phenylamino)-2-thiophen-2-ylimidazo[1,2-a]pyrimidin-1-iumchlorid
1-Acetyl-3-cyclohexylamino-2-furan-2-yl-imidazo[1,2-a]pyridin-1-iumchlorid Hydrochlorid

Soweit die erfindungsgemäßen Salze bicyclischer, N-acylierter Imidazo-3-amine und Imidazo-5-amine optisch aktive Kohlenstoffatome enthalten, sind auch die Enantiomeren dieser Verbindungen und deren Mischungen Gegenstand der vorliegenden Erfindung.

Unter einem Aryl-Rest wird vorzugsweise ein gegebenenfalls ein- oder mehrfach substituierter Phenyl- oder Naphthyl-Rest verstanden.

Unter einem Heteroaryl-Rest werden aromatische Reste verstanden, die wenigstens ein Heteroatom, vorzugsweise Stickstoff, Sauerstoff und/oder Schwefel, besonders bevorzugt Stickstoff und/oder Sauerstoff aufweisen.

Überraschenderweise wurde darüber hinaus gefunden, daß die erfindungsgemäßen Salze bicyclischer, N-acylierter Imidazo-3-amine und Imidazo-5-amine an den µ-Opiatrezeptor binden und sich daher auch als pharmazeutische Wirkstoffe eignen.

Gegenstand der Erfindung sind daher auch Arzneimittel enthaltend als Wirkstoff mindestens ein Salz eines bicyclischen, N-acylierten Imidazo-3-amins oder Imidazo-5-amins der allgemeinen Formel I, in der R¹ bis R⁸ und A die oben angegebene Bedeutung haben und X für das Anion einer pharmazeutisch akzeptablen anorganischen oder organischen Säure, vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure oder insbesondere der Salzsäure steht, und/oder dessen Additionsprodukt mit einer physiologisch verträglichen Säure, vorzugsweise der Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure.

Bevorzugt enthält dabei das erfindungsgemäße Arzneimittel als Wirkstoff mindestens ein Salz eines bicyclischen, N-acylierten Imidazo-3-amins oder Imidazo-5-amins oder dessen Säureadditionsprodukt ausgewählt aus der Gruppe
1-Acetyl-3-*tert*-butylamino-2-phenyl-imidazo[1,2-a]-pyridin-1-iumchlorid
1-Acetyl-3-*tert*-butylamino-2-phenyl-imidazo[1,2-a]-pyrazin-1-iumchlorid
1-Acetyl-3-*tert*-butylamino-2-phenyl-imidazo[1,2-a]-pyrimidin-1-iumchlorid
7-Acetyl-5-*tert*-butylamino-6-phenyl-imidazo[2,1-b]-thiazol-7-iumchlorid
3-Acetyl-1-cyclohexylamino-2-phenyl-imidazo[1,2-a]-chinolin-3-iumchlorid
1-Acetyl-3-cyclohexylamino-2-phenyl-imidazo[1,2-a]-pyrimidin-1-iumchlorid
1-Acetyl-8-benzyloxy-3-cyclohexylamino-2-methylimidazo[1,2-a]pyridin-1-iumchlorid
1-Acetyl-8-benzyloxy-3-tert-butylamino-2-methylimidazo[1,2-a]pyridin-1-iumchlorid
1-Acetyl-8-benzyloxy-2-methyl-3-(1,1,3,3-tetramethylbutylamino)imidazo[1,2-a]pyridin-1-iumchlorid
3-(*tert*-Butyl-cyclohexancarbonyl-amino)-1-cyclohexancarbonyl-2-phenyl-imidazo[1,2-a]pyridin-1-iumchlorid
3-(*tert*-Butyl-heptanoyl-amino)-1-heptanoyl-2-phenylimidazo[1,2-a]pyridin-1-iumchlorid
1-Acetyl-3-cyclohexylamino-2-furan-2-yl-imidazo[1,2-a]-pyridin-1-iumchlorid
1-Acetyl-3-cyclohexylamino-2-furan-2-yl-imidazo[1,2-a]-pyrimidin-1-iumchlorid
1-Acetyl-5-amino-7-chlor-3-cyclohexylamino-2-furan-2-ylimidazo[1,2-a]pyrimidin-1-iumchlorid
1-Acetyl-3-cyclohexylamino-2-pyridin-2-yl-imidazo-[1,2-a]pyridin-1-iumchlorid
1-Acetyl-3-cyclohexylamino-6-nitro-2-pyridin-2-ylimidazo[1,2-a]pyridin-1-iumchlorid
1-Acetyl-3-cyclohexylamino-5-methyl-2-pyridin-2-ylimidazo[1,2-a]pyridin-1-iumchlorid
1-Acetyl-3-cyclohexylamino-2-pyridin-2-yl-imidazo[1,2-a]pyrazin-1-iumchlorid
1-Acetyl-3-cyclohexylamino-2-pyridin-2-yl-imidazo-[1,2-a]pyrimidin-1-iumchlorid
1-Acetyl-5-amino-7-chlor-3-cyclohexylamino-2-pyridin-2-ylimidazo[1,2-a]pyrimidin-1-iumchlorid
1-Acetyl-3-cyclohexylamino-5,7-dimethyl-2-pyridin-2-ylimidazo[1,2-a]pyrimidin-1-iumchlorid
7-Acetyl-5-cyclohexylamino-6-pyridin-2-yl-imidazo-[2,1-b]thiazol-7-iumchlorid
1-Acetyl-2-cyclohexyl-3-cyclohexylamino-imidazo[1,2-a]-pyridin-1-iumchlorid
1-Acetyl-3-cyclohexylamino-2-methyl-imidazo[1,2-a]-pyrimidin-1-iumchlorid
1-Acetyl-5-amino-7-chlor-3-cyclohexylamino-2-methylimidazo[1,2-a]pyrimidin-1-iumchlorid
1-Acetyl-5-amino-7-chlor-3-cyclohexylamino-2-thiophen-2-yl-imidazo[1,2-a]pyrimidin-1-iumchlorid
1-Acetyl-5-amino-7-chlor-2-furan-2-yl-3-(methoxycarbonylmethylamino)imidazo[1,2-a]pyrimidin-1-iumchlorid
1-Acetyl-2-cyclohexyl-3-(methoxycarbonylmethylamino)-imidazo[1,2-a]pyridin-1-iumchlorid
1-Acetyl-2-cyclohexyl-3-(methoxycarbonylmethylamino)-imidazo[1,2-a]pyrimidin-1-iumchlorid
1-Acetyl-3-(2,6-dimethyl-phenylamino)-2-methyl-6-nitroimidazo[1,2-a]pyridin-1-iumchlorid
1-Acetyl-5-amino-7-chlor-3-(2,6-dimethyl-phenylamino)-2-thiophen-2-ylimidazo[1,2-a]pyrimidin-1-iumchlorid
1-Acetyl-3-cyclohexylamino-2-furan-2-yl-imidazo[1,2-a]pyridin-1-iumchlorid Hydrochlorid

Dabei zeigen die erfindungsgemäßen Salze insbesondere eine analgetische Wirkung. Eine besonders bevorzugte Ausführungsform der Erfindung ist daher die Verwendung von Salzen mindestens eines bicyclischen, N-acylierten Imidazo-3-amins oder Imidazo-5-amins der allgemeinen Formel I, in der R¹ bis R⁸, A und X die oben angegebene Bedeutung haben, und/oder von deren Additionsprodukten mit einer physiologisch verträglichen Säure zur Herstellung eines Arzneimittels zur Behandlung von Schmerz.

Darüber hinaus eignen sich diese Wirkstoffe insbesondere zur Behandlung von Drogen- und/oder Alkoholmißbrauch, Diarrhoe, Gastritis, Ulcera, Harninkontinenz, Depressionen, Narkolepsie, Übergewicht, Asthma, Glaukom, Tinnitus, Juckreiz und/oder hyperkinetischem Syndrom. Sie eignen sich außerdem zur Behandlung/Prophylaxe von/bei Epilepsie und Schizophrenie, und/oder zur Anxiolyse und/oder Anästhesie.

Die erfindungsgemäßen Arzneimittel, insbesonderere Schmerzmittel (Analgetika) enthalten neben mindestens einem Salz der Formel I oder dessen Additionsprodukt mit einer physiologisch verträglichen Säure Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben erfolgt in der dem Fachmann bekannten Weise und hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Salze der Formel I oder deren Additionsprodukte mit physiologisch verträglichen Säuren in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Salze der Formel I oder deren Additionsprodukte mit physiologisch verträglichen Säuren verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 2 bis 500 mg/kg der Salze von bicyclischen, N-acylierten Imidazo-3-aminen oder Imidazo-5-aminen der Formel **I** oder ihrer Additionsprodukte mit physiologisch verträglichen Säuren appliziert.

Weiterer Gegenstand der Erfindung sind Verfahren zur Herstellung der Salze von bicyclischen, N-acylierten Imidazo-3-aminen und Imidazo-5-aminen der Formel I und ihrer Additionsprodukte mit Säuren. Diese Verfahren umfassen folgende Verfahrensschritte:
a) Herstellung der Imidazo-3-amine bzw. Imidazo-5-amine durch Dreikomponentenreaktion aus Amidin, Aldehyd und Isonitril in einem Lösungsmittel, vorzugsweise Dichlormethan, und in Gegenwart einer Säure, vorzugsweise Perchlorsäure, wobei die Ausgangsverbindungen nacheinander in der Reihenfolge Amidin, Aldehyd und Isonitril zugegeben werden,
b) gegebenenfalls zur Herstellung von Verbindungen, bei denen R² nicht Wasserstoff bedeutet, Umsetzung der in Schritt a) entstehenden Produkte mit einer Verbindung R²Hal (Hal = Brom, Iod oder insbesondere Chlor) oder einem Isocyanat,
c) Umsetzung des Reaktionsprodukts aus Schritt a) bzw. b) mit einem vorzugsweise mindestens vierfachen molaren Überschuß eines Säurechlorids R⁸C(O)Cl,
d) nach Reaktionsende Entfernung des überschüssigen Säurechlorids aus dem Reaktionsgemisch,
e) Gegebenenfalls in an sich bekannter Weise Austausch des Chlorids gegen einen anderen Säurerest vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure oder Asparaginsäure.
f) Gegebenenfalls Herstellung des erfindungsgemäßen Additionsproduktes mit einer vorzugsweise physiologisch verträglichen Säure.

Verfahrensschritt a) erfolgt vorzugsweise so, daß man Amidine mit der allgemeinen Formel II, insbesondere 3-Aminopyrazol-, 3-Amino-1,2,4-triazol-, 2-Amino-1,3,4-thiadiazol-, 2-Aminothiazol-, 2-Aminopyridin-, 2-Aminopyrimidin- und 2-Aminopyrazinderivate, die von Firmen wie beispielsweise Acros, Avocado, Aldrich, Fluka, Lancaster, Maybridge, Merck, Sigma oder TCI-Jp kommerziell angeboten werden, mit verschiedensten Aldehyden **III** und Isonitrilen **IV** in Gegenwart von 20%iger Perchlorsäure gemäß einer Dreikomponentenreaktion zu Verbindungen der Formel **V** umsetzt. R1 und R3 sowie A haben dabei die oben für Verbindungen der Formel **I** angegebene Bedeutung.

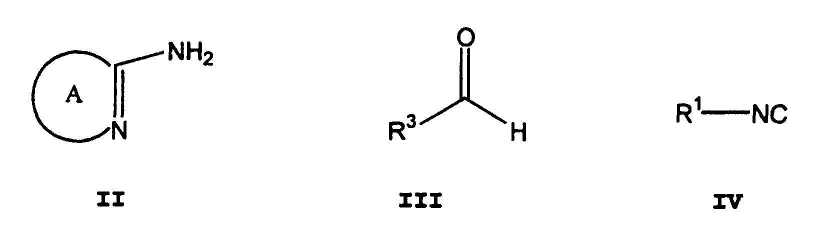

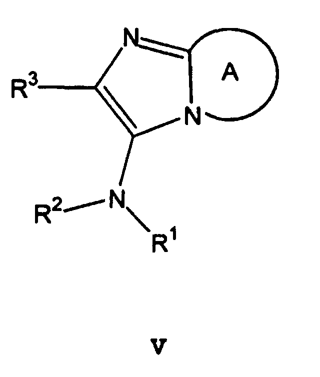

Der Reaktionsschritt a) wird in einem Lösungsmittel, vorzugsweise Dichlormethan (DCM), bei einer Temperatur von -20°C bis 100°C, bevorzugt bei 0°C bis 40°C und ganz besonders bevorzugt bei 10°C bis 20°C durchgeführt.

Zur Herstellung der erfindungsgemäßen Verbindungen, in denen R² nicht Wasserstoff bedeutet, wurden gegebenenfalls die in Reaktionsschritt a) entstehenden Verbindungen **V** in einem Lösungsmittel, vorzugsweise THF oder DCM, gelöst, je nach gewünschtem Endprodukt mit einer Verbindung R²Hal, wobei Hal für Brom, Iod oder insbesondere Chlor steht, beispielsweise einem gegebenenfalls substituierten Alkyl-, Aryl- oder Säurechlorid, oder, falls R² CONHR⁸ wie oben angegeben bedeuten soll, mit einem Isocyanat in Gegenwart einer anorganischen oder organischen Base, bevorzugt in Gegenwart eines Morpholin-Harzes (z.B. Polystyrol-Morpholin der Firma Argonaut), in einem Lösungsmittel, vorzugsweise Dichlormethan, innerhalb von 2 bis 24 Stunden bei Temperaturen zwischen -20°C und 100°C, bevorzugt zwischen 10°C und 40°C, zu Verbindungen der Formel **VI** umgesetzt, in denen R² nicht Wasserstoff bedeutet:

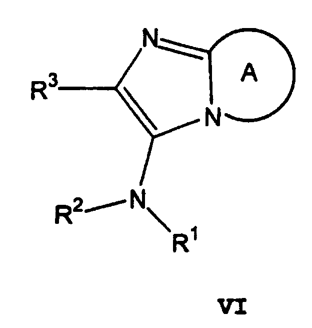

Anschließend wird in Reaktionsschritt c) das Reaktionsprodukt **V** aus Reaktionsschritt a) oder das Reaktionsprodukt **VI** aus Reaktionsschritt b), bei dem R² nicht Wasserstoff bedeutet, mit einem Überschuß, vorzugsweise einem mindestens vierfachen, insbesondere einem 4 bis 10fachen Überschuß eines Säurechlorids R⁸C(O)Cl zu dem weiter unten angegebenen erfindungsgemäßen Salz der Formel **Ia** umgesetzt, wobei R⁸ die oben für die Formel **I** angegebene Bedeutung hat. Dieser Reaktionsschritt wird in einem Lösungsmittel, vorzugsweise in einem Ether oder Halogenkohlenwasserstoff, besonders bevorzugt in THF oder DCM, bei einer Temperatur zwischen -20°C und 100°C, vorzugsweise zwischen 0 °C und 60 °C durchgeführt.

Wird ein Reaktionsprodukt V aus Reaktionsschritt a) auf diese Weise umgesetzt, kann auch in diesem Schritt ein erfindungsgemäßes Produkt der Formel **I** erhalten werden, bei dem R² kein Wasserstoffatom, sondern ein Säurerest COR^{b} ist, wobei R^{b} identisch mit R⁸ ist. In Abhängigkeit von der Reaktionsführung kann dieses Produkt ein Nebenprodukt oder auch Hauptprodukt bzw. alleiniges Produkt der Reaktion sein. Zur gezielten Darstellung dieses Produktes wird vorzugsweise mit einem sehr großen, insbesondere mindestens zehnfachen Überschuß des Säurechlorids und/oder bei hoher Temperatur und/oder mit langer Reaktionsdauer gearbeitet. Eventuell erhaltene Produktgemische lassen sich mit bekannten Verfahren, beispielsweise durch Chromatographie oder vorzugsweise in Verfahrensschritt f) durch Fällung des Additionsprodukts mit einer Säure aus einem Lösungsmittel oder Lösungsmittelgemisch trennen.

Vorzugsweise 2 bis 12 Stunden nach Zugabe des Säurechlorids in Verfahrensschritt c) wird in Verfahrensschritt d) das überschüssige Säurechlorid aus dem Reaktionsgemische entfernt. Dies kann grundsätzlich mittels der üblichen, dem Fachmann bekannten Methoden geschehen, beispielsweise im Vakuum oder durch Verwendung anorganischer oder organischer Basen. Erfindungsgemäß bevorzugt ist es jedoch, diese Trennung in heterogener Phase, insbesondere durch Zugabe eines Scavanger-Harzes vorzunehmen. Als Scavanger-Harz wird dabei vorzugsweise (Tris-(2-Aminoethyl)amin-polystyrol eingesetzt. Beispiele für die besonders bevorzugte Ausführungsform der Verfahrensschritte c) und d) gibt das folgende Reaktionsschema wieder:

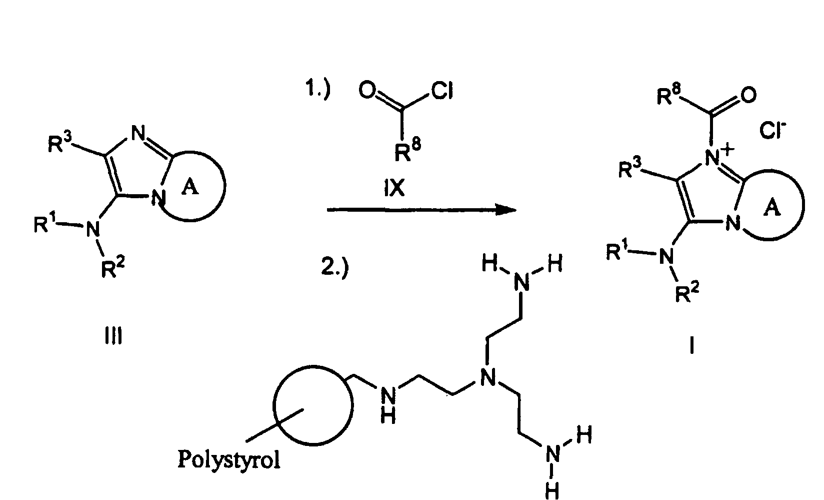

Zur Herstellung von erfindungsgemäßen Salzen der Formel **I**, in denen X einen anderen Säurerest als Chlorid bedeutet, erfolgt gegebenenfalls in Verfahrensschritt e) in an sich bekannter Weise der Austausch des Chlorids gegen den Rest einer anderen organischen oder anorganischen Säure, vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure oder Asparaginsäure. Bevorzugt werden hierbei entsprechend präparierte basische Ionenaustauscher verwendet

Das erfindungsgemäße Verfahren ist insbesondere auch zur Durchführung in automatischen Syntheseanlagen geeignet.

Zur gezielten Darstellung von erfindungsgemäßen Verbindungen der Formel **I** aus Reaktionsprodukten V aus Reaktionsschritt a), für die also R² ein Wasserstoffatom darstellt, können zusätzlich Schutzgruppenstrategien angewendet werden (P.J. Kocienski, Protecting Groups, Thieme Verlag, 1994; T.W. Greene, P.G.M. Wuts, Protective Groups in Organic Synthesis, John Wiley & Sons, 3.Aufl., 1999). Dazu wird vorzugsweise die Gruppierung R²N in ein Carbamat, besonders bevorzugt in ein Benzyl- oder tert.-Butylcarbamat, oder ein Amid oder R² in eine Silylgruppe, vorzugsweise tert.-Butyldimethylsilyl oder Trimethylsilyl überführt. Diese Schutzgruppen können gegebenenfalls nach der Umsetzung mit einem Säurechlorid R⁸C(O)Cl in bekannter Weise entfernt werden.

Verfahrensschritt f) erfolgt vorzugsweise durch Umsetzung des Reaktionsprodukts aus Schritt d) bzw. e) mit physiologisch verträglichen Säuren, vorzugsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in einem Lösungsmittel oder Lösungsmittelgemisch, vorzugsweise Diethylether, Diisopropylether, Essigsäurealkylester, Aceton und/oder 2-Butanon, wobei das entstehende Additionsprodukt ausfällt, gegebenenfalls nach Entfernung eines Teils des Lösungsmittels oder Zugabe eines weiteren, unpolareren Lösungsmittels, beispielsweise eines Kohlenwasserstoffs oder aliphatischen Ethers. Besonders bevorzugt ist dabei zur Herstellung des HCl-Additionsprodukts die Umsetzung mit einer wässrigen Lösung von Trimethylchlorsilan. Verfahrensschritt f) wird erfindungsgemäß bevorzugt auch zur Aufreinigung der Salze gemäß der allgemeinen Formel I eingesetzt. In diesem Fall wird das Salz nach der oben beschriebenen Fällung wieder in üblicher Weise aus dem Additionsprodukt freigesetzt.

Sofern ein Salz der Formel I unter Anwendung des Verfahrensschritts d) hergestellt wird und Verfahrensschritt f) zur Aufreinigung zur Anwendung kommt, kann dieser alternativ oder auch zusätzlich vor Verfahrensschritt d) durchgeführt werden.

### Beispiele:

Die folgenden Beispiele sollen die Erfindung erläutern, ohne sie darauf zu beschränken.

### Allgemeine Synthesevorschrift für die Imidazole der Formel V (Verfahrensschritt a)):

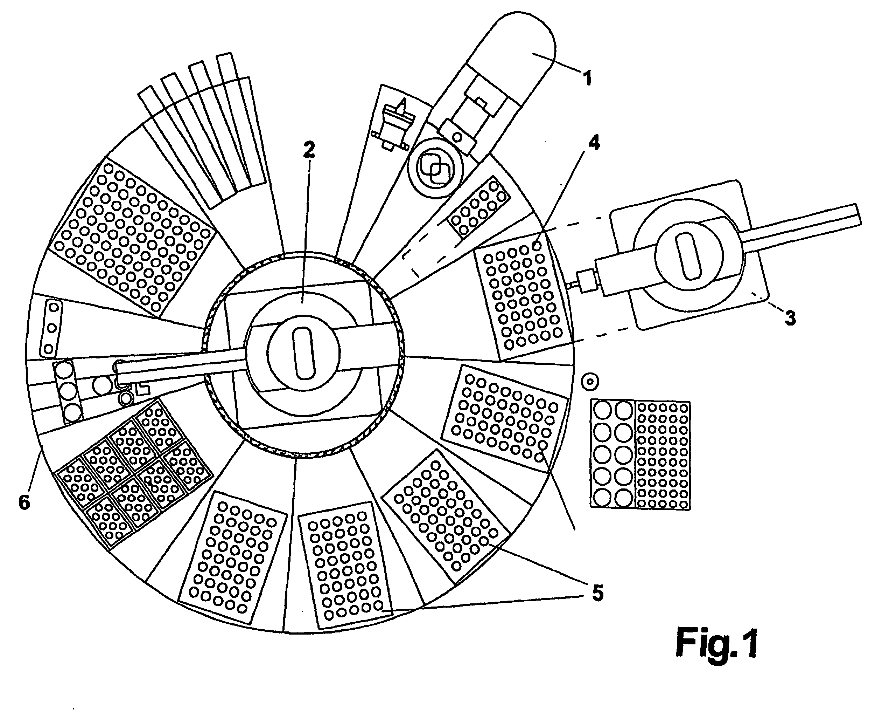

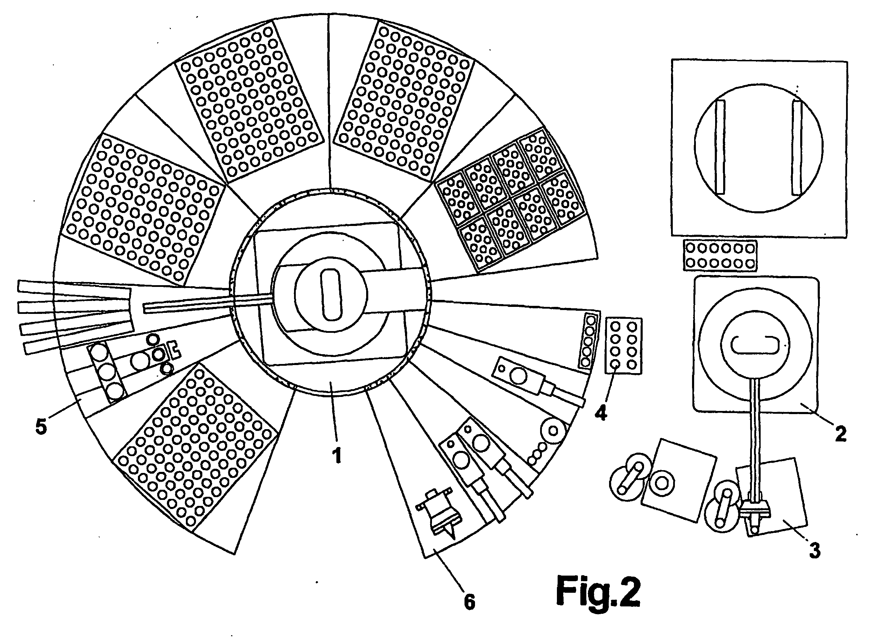

Die Synthese der Imidazole der Formel V erfolgte auf einer automatischen Anlage der Firma Zymark gemäß Figur 1 bzw. Figur 2.

Dabei umfaßt Figur 1 eine Capper-Station (Ziff. 1) zum Verschließen der Reaktionsröhrchen, einen Roboter 1 (Ziff. 2) und einen Roboter 2 (Ziff. 3), wobei der Roboter 1 die Reaktionsröhrchen bzw. die entsprechenden Racks bewegt und der Roboter 2 die Reagenzien in die Reaktionsröhrchen pipettiert, einen temperierbaren Reaktorblock (Ziff. 4), Rührblöcke (Ziff. 5) und eine Filtrationsstation (Ziff. 6), in der die Reaktionslösung abfiltriert wird.

Figur 2 umfaßt ebenfalls einen Roboter 1 (Ziff. 1) und einen Roboter 2 (Ziff. 2), die beide die Glasröhrchen mit den Syntheseprodukten auf die verschiedenen Stationen bewegen. Bei den Stationen handelt es sich im einzelnen um einen Vortexer (Ziff. 3) zum Durchmischen der Proben und zum Zudosieren von Lösungen oder Lösungsmitteln, einen Spin-Reaktor (Ziff. 4) zur Durchmischung von Proben, eine Phasendetektionsstation (Ziff. 5) zur Detektion der Phasengrenze und Phasentrennung sowie eine Station (Ziff. 6) zum Trocknen der Syntheseprodukte über Salzkartuschen.

Die Synthese erfolgte nach folgender allgemeinen Vorschrift:

Ein Rundbodenröhrchen aus Glas (Durchmesser 16 mm, Länge 125 mm) mit Gewinde wurde manuell mit einem Rührer versehen und auf der Capper-Station mit einem Schraubdeckel mit Septum verschlossen. Das Röhrchen wurde von Roboter 1 in den auf 15°C temperierten Reaktorblock gestellt. Roboter 2 pipettierte nacheinander folgende Reagenzien hinzu:
1.) 1 ml einer 0,1 M Amidin-Lösung + 20% HClO₄ in Dichlormethan
2.) 0,5 ml einer 0,3 M Aldehyd-Lösung in Dichlormethan
3.) 0,575 ml einer 0,2 M Isonitril-Lösung in Dichlormethan

Das Reaktionsgemisch wurde bei 15°C in einem der Rührblöcke 660 min lang gerührt. Danach wurde die Reaktionslösung an der Filtrations-Station abfiltriert. Das Röhrchen wurde dabei zweimal mit je 1 ml Dichlormethan und 200 µl Wasser gespült.

Das Rack mit den Röhrchen wurde anschließend manuell auf die Aufarbeitungsanlage (Figur 2) gestellt. Dort wurde das Reaktionsgemisch auf einem Vortexer mit 3 ml einer 10%igen NaCl-Lösung und 1,5 ml Dichlormethan versetzt. Im Spin-Reaktor wurde zehn Minuten lang gründlich gemischt und durch die langsame Abnahme der Drehbewegung eine deutliche Phasengrenze ausgebildet. Diese Phasengrenze wurde optisch detektiert und die organische Phase abpipettiert. Im nächsten Schritt wurde das Reaktionsgemisch erneut mit 1,5 ml Dichlormethan versetzt. Die Lösung wurde geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

### Allgemeine Vorschrift für die Verfahrensschritte c) und d) :

Die nachfolgenden Beispiele der erfindungsgemäßen Salze der Formel **I** aus Imidazolen der Formel **V** (Verfahrensschritte c) und d))wurden auf einem Syntheseroboter der Firma MultiSyntech synthetisiert. Der Syntheseroboter umfasst einen heizund kühlbaren Reaktionsblock mit 40 Rührpositionen, ein Reagenzienrack mit 32 Positionen, sieben größere Gefäße für Reagenzien sowie zwei Pipettierarme zur Zugabe von Lösungsmitteln und Reagenzien. Die erfindungsgemäßen Salze der Formel I wurden nach der folgenden allgemeinen Vorschrift hergestellt:

In einem Glasröhrchen, das zuvor manuell in den Reaktorblock gestellt wurde, wurden 0,1 mmol des Imidazols pipettiert. Nach Zugabe von 2ml THF und ca. 10 min. Rühren werden 0,4 mmol des Säurechlorids zugegeben. Das Reaktionsgemisch wird 6 h bei Raumtemperatur gerührt. Im folgenden Schritt werden manuell 0,5 mmol des Scavenger-Harzes (Tris-(2-Aminoethyl)amin-polystyrol, 2,43 mmol/g) zugegeben. Nach 2 h Rühren wurde die Reaktionslösung vom Harz durch filtrieren abgetrennt, dreimal mit je 1,5 ml Dichlormethan nachgewaschen und in einer Vakuumzentrifuge eingeengt.

Soweit die Herstellung hier nicht beschrieben ist, wurden die eingesetzten Chemikalien und Lösungsmittel kommerziell bei den herkömmlichen Anbietern erworben (Acros, Avocado, Aldrich, Fluka, Lancaster, Maybridge, Merck, Sigma, TCI-Jp, Novabiochem). Alle Produkte werden mit NMR oder ESI-MS analysiert.

### Beispiel 1

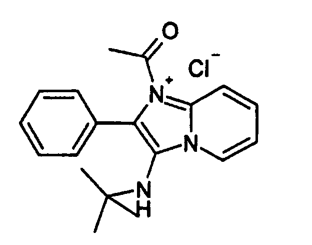

### 1-Acetyl-3-tert-butylamino-2-phenyl-imidazo[1,2-a]-pyridin-1-iumchlorid

Beispiel 1 wurde gemäß der allgemeinen Synthesevorschrift im Verfahrensschritt a) aus 1.0 ml (0.1 mmol) 2-Amino-pyridin (0.1M, DCM), 0.575 ml (0.115 mmol) tert.-Butylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 308,41, gefundene Masse M⁺ = 308,1

### Beispiel 2

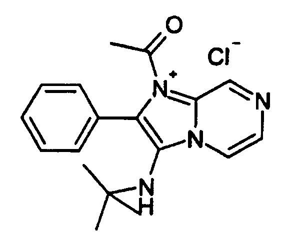

### 1-Acetyl-3-tert-butylamino-2-phenyl-imidazo[1,2-a]-pyrazin-1-iumchlorid

Beispiel 2 wurde gemäß der allgemeinen Synthesevorschrift im Verfahrensschritt a) aus 1.0 ml (0.1 mmol) 2-Amino-pyrazin (0.1M, DCM), 0.575 ml (0.115 mmol) tert.-Butylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 309,35 gefundene Masse M⁺ = 309,2

### Beispiel 3

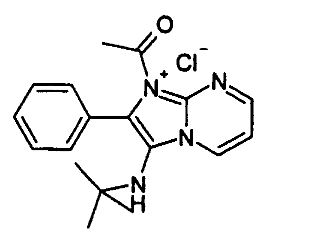

### 1-Acetyl-3-tert-butylamino-2-phenyl-imidazo[1,2-a]-pyrimidin-1-iumchlorid

Beispiel 3 wurde gemäß der allgemeinen Synthesevorschrift im Verfahrensschritt a) aus 1.0 ml (0.1 mmol) 2-Amino-pyrimidin (0.1M, DCM), 0.575 ml (0.115 mmol) tert.-Butylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.
Charakterisierung durch ESI-MS, berechnete Masse M = 309,35, gefundene Masse M⁺ = 309,2

### Beispiel 4

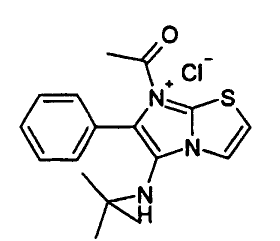

### 7-Acetyl-5-tert-butylamino-6-phenyl-imidazo[2,1-b]-thiazol-7-iumchlorid

Beispiel 4 wurde gemäß der allgemeinen Synthesevorschrift im Verfahrensschritt a) aus 1.0 ml (0.1 mmol) 2-Aminothiazol (0.1M, DCM), 0.575 ml (0.115 mmol) tert.-Butylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 314,39, gefundene Masse M⁺ = 314,1

### Beispiel 5

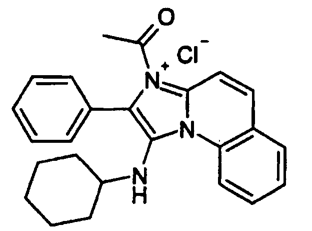

### 3-Acetyl-1-cyclohexylamino-2-phenyl-imidazo[1,2-a]-chinolin-3-iumchlorid

Beispiel 5 wurde gemäß der allgemeinen Synthesevorschrift im Verfahrensschritt a) aus 1.0 ml (0.1 mmol) 2-Aminochinolin (0.1M, DCM), 0.575 ml (0.115 mmol) Cyclohexylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 419,96, gefundene Masse M-H = 418,5

### Beispiel 6

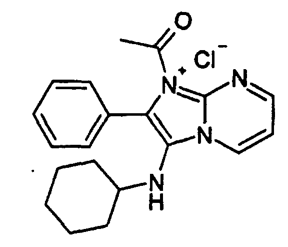

### 1-Acetyl-3-cyclohexylamino-2-phenyl-imidazo[1,2-a]-pyrimidin-1-iumchlorid

Beispiel 6 wurde gemäß der allgemeinen Synthesevorschrift im Verfahrensschritt a) aus 1.0 ml (0.1 mmol) 2-Amino-pyrimidin (0.1M, DCM), 0.575 ml (0.115 mmol) Cyclohexylisonitril-Lösung (0.2M, DCM), 0.500 ml (0.15 mmol) Benzaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 335,39, gefundene Masse M⁺ = 335,3

### Beispiel 7

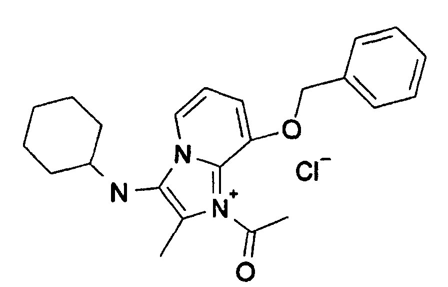

### 1-Acetyl-8-benzyloxy-3-cyclohexylamino-2-methylimidazo[1,2-a]pyridin-1-iumchlorid

Beispiel 7 wurde gemäß der allgemeinen Synthesevorschrift im Verfahrensschritt a) aus 1.0 ml (0.1 mmol) 2-Amino-3-benzyloxypyridin (0.1M, DCM), 0.575 ml (0.115 mmol) Cyclohexylisonitril-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 378,5, gefundene Masse M⁺ - Acetyl = 336,4

### Beispiel 8

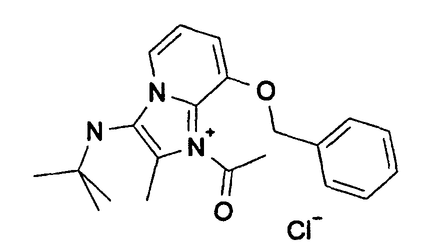

### 1-Acetyl-8-benzyloxy-3-tert-butylamino-2-methylimidazo[1,2-a]pyridin-1-iumchlorid

Beispiel 8 wurde gemäß der allgemeinen Synthesevorschrift im Verfahrensschritt a) aus 1.0 ml (0.1 mmol) 2-Amino-3-benzyloxypyridin (0.1M, DCM), 0.575 ml (0.115 mmol) tert-Butylisonitril-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 352,46, gefundene Masse M⁺ - Acetyl = 310,3

### Beispiel 9

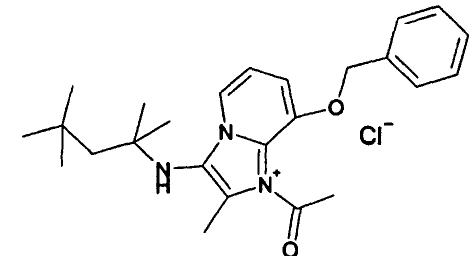

### 1-Acetyl-8-benzyloxy-2-methyl-3-(1,1,3,3-tetramethylbutylamino)imidazo[1,2-a]pyridin-1-iumchlorid

Beispiel **9** wurde gemäß der allgemeinen Synthesevorschrift im Verfahrensschritt a) aus 1.0 ml (0.1 mmol) 2-Amino-3-benzyloxypyridin (0.1M, DCM), 0.575 ml (0.115 mmol) 1,1,3,3-Tetramethylbutylisonitril-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M = 408,57, gefundene Masse M⁺ - Acetyl = 366,0

### Beispiel 10

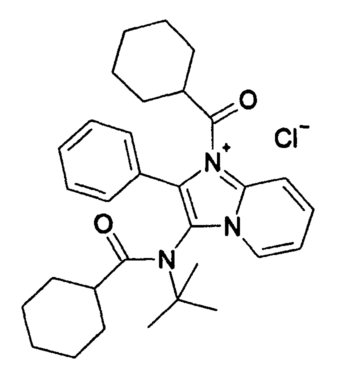

### 3-(tert-Butyl-cyclohexancarbonyl-amino)-1-cyclohexancarbonyl-2-phenyl-imidazo[1,2-a]pyridin-1-iumchlorid

Beispiel **10** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) *tert*-Butylisonitril-Lösung (0.2M, DCM), 0.500 ml (0.15 mmol) Benzaldehyd-Lösung (0.3 M, DCM) und 10 µl Perchlorsäure (w = 20%) und in den Verfahrensschritten b) bis d) durch Umsetzung des erhaltenen Reaktionsprodukts mit 0,4 mmol Cyclohexylcarbonsäurechlorid dargestellt.

Charakterisierung durch ESI-MS, berechnete Masse M - Cl⁻ = 486,68, gefundene Masse M⁺ = 486,3

### Beispiel 11

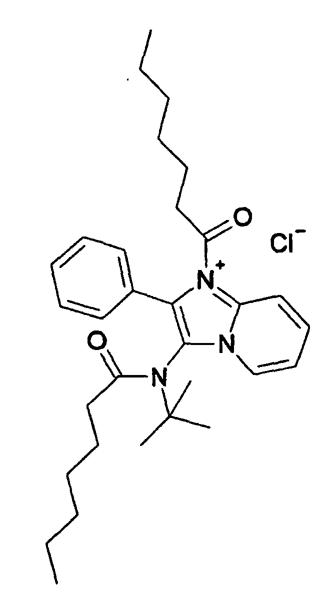

### 3-(tert-Butyl-heptanoyl-amino)-1-heptanoyl-2-phenylimidazo[1,2-a]pyridin-1-iumchlorid

Beispiel **11** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) tert-Butylisonitril-Lösung (0.2M, DCM), 0.500 ml (0.15 mmol) Benzaldehyd-Lösung (0.3 M, DCM) und 10 µl Perchlorsäure (w = 20%) und in den Verfahrensschritten b) bis d) durch Umsetzung des erhaltenen Reaktionsprodukts mit 0,4 mmol Heptylcarbonsäurechlorid dargestellt.

Charakterisierung durch ESI-MS, berechnete Masse M - Cl⁻ = 490,72, gefundene Masse M⁺ = 490,3

### Beispiel 12

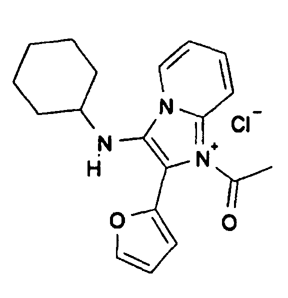

### 1-Acetyl-3-cyclohexylamino-2-furan-2-yl-imidazo[1,2-a]-pyridin-1-iumchlorid

Beispiel **12** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) Cyclohexylisonitril-Lösung (0.2M, DCM), 0.500 ml (0.15 mmol) Furfural-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 324,41, gefundene Masse M⁺ = 324,3

### Beispiel 13

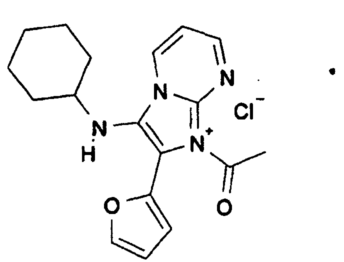

### 1-Acetyl-3-cyclohexylamino-2-furan-2-yl-imidazo[1,2-a]-pyrimidin-1-iumchlorid

Beispiel **13** wurde gemäß der allgemeinen Synthesevorschrift im Verfahrensschritt a) aus 1.0 ml (0.1 mmol) 2-Aminopyrimidin (0.1M, DCM), 0.575 ml (0.115 mmol) Cyclohexylisonitril-Lösung (0.2M, DCM), 0.500 ml (0.15 mmol) Furfural-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 325,39, gefundene Masse M⁺ = 325,3

### Beispiel 14

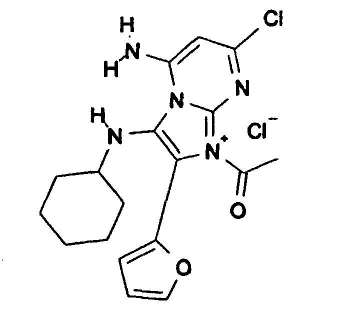

### 1-Acetyl-5-amino-7-chlor-3-cyclohexylamino-2-furan-2-ylimidazo[1,2-a]pyrimidin-1-iumchlorid

Beispiel **14** wurde gemäß der allgemeinen Synthesevorschrift im Verfahrensschritt a) aus 1.0 ml (0.1 mmol) 2,6-Diamino-4-chlorpyrimidin (0.1M, DCM), 0.575 ml (0.115 mmol) Cyclohexylisonitril-Lösung (0.2M, DCM), 0.500 ml (0.15 mmol) Furfural-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 374,85, gefundene Masse M⁺ = 374,5

### Beispiel 15

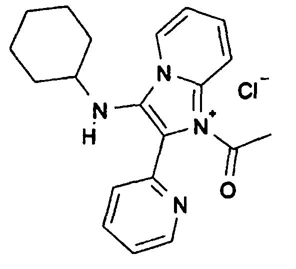

### 1-Acetyl-3-cyclohexylamino-2-pyridin-2-yl-imidazo-[1,2-a]pyridin-1-iumchlorid

Beispiel **15** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 335,42, gefundene Masse M⁺ = 335,4

### Beispiel 16

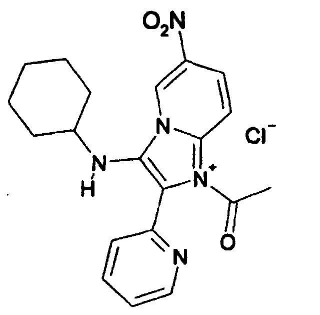

### 1-Acetyl-3-cyclohexylamino-6-nitro-2-pyridin-2-ylimidazo[1,2-a]pyridin-1-iumchlorid

Beispiel 16 wurde gemäß der allgemeinen Synthesevorschrift im Verfahrensschritt a) aus 1.0 ml (0.1 mmol) 2-Amino-5-nitropyridin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 380,43, gefundene Masse M⁺ = 380,3

### Beispiel 17

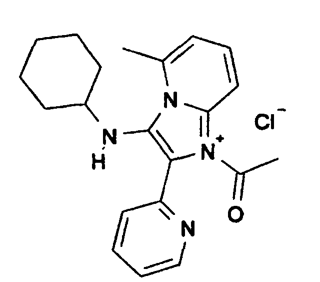

### 1-Acetyl-3-cyclohexylamino-5-methyl-2-pyridin-2-ylimidazo[1,2-a]pyridin-1-iumchlorid

Beispiel **17** wurde gemäß der allgemeinen Synthesevorschrift im Verfahrensschritt a) aus 1.0 ml (0.1 mmol) 2-Amino-6-methylpyridin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 349,46, gefundene Masse M⁺ = 349,4

### Beispiel 18

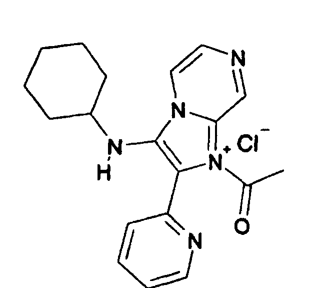

### 1-Acetyl-3-cyclohexylamino-2-pyridin-2-yl-imidazo[1,2-a]pyrazin-1-iumchlorid

Beispiel **18** wurde gemäß der allgemeinen Synthesevorschrift im Verfahrensschritt a) aus 1.0 ml (0.1 mmol) 2-Aminopyrazin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 336,42, gefundene Masse M⁺ = 336,3

### Beispiel 19

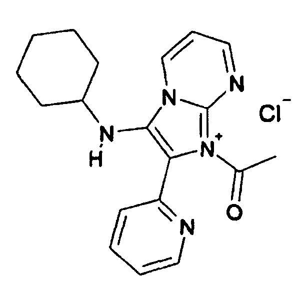

### 1-Acetyl-3-cyclohexylamino-2-pyridin-2-yl-imidazo-[1,2-a]pyrimidin-1-iumchlorid

Beispiel **19** wurde gemäß der allgemeinen Synthesevorschrift im Verfahrensschritt a) aus 1.0 ml (0.1 mmol) 2-Aminopyrimidin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 336,42, gefundene Masse M⁺ = 336,3

### Beispiel 20

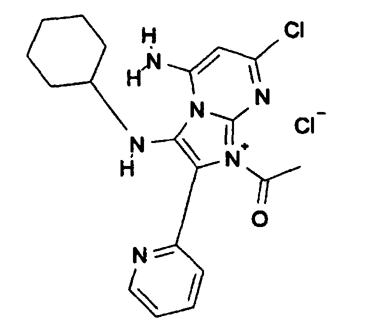

### 1-Acetyl-5-amino-7-chlor-3-cyclohexylamino-2-pyridin-2-ylimidazo[1,2-a]pyrimidin-1-iumchlorid

Beispiel 20 wurde gemäß der allgemeinen Synthesevorschrift im Verfahrensschritt a) aus 1.0 ml (0.1 mmol) 2,6-Diamino-4-chlor-pyrimidin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 336,42, gefundene Masse M⁺ = 336,3

### Beispiel 21

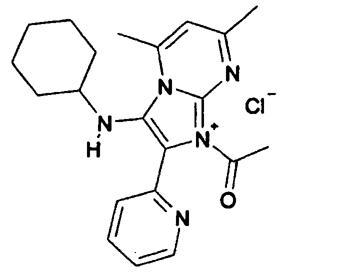

### 1-Acetyl-3-cyclohexylamino-5,7-dimethyl-2-pyridin-2-ylimidazo[1,2-a]pyrimidin-1-iumchlorid

Beispiel **21** wurde gemäß der allgemeinen Synthesevorschrift im Verfahrensschritt a) aus 1.0 ml (0.1 mmol) 2-Amino-4,6-dimethylpyrimidin (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 364,47, gefundene Masse M⁺ = 364,4

### Beispiel 22

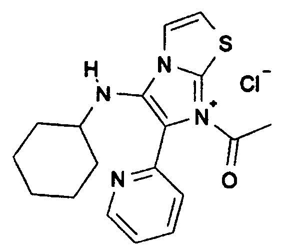

### 7-Acetyl-5-cyclohexylamino-6-pyridin-2-yl-imidazo-[2,1-b]thiazol-7-iumchlorid

Beispiel **22** wurde gemäß der allgemeinen Synthesevorschrift im Verfahrensschritt a) aus 1.0 ml (0.1 mmol) 2-Aminothiazol (0.1M, DCM), 0.575 ml (0.115 mmol) Cyclohexylisonitril-Lösung (0.2M, DCM), 0.500 ml (0.15 mmol) Pyridin-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 341,46, gefundene Masse M⁺ = 341,2

### Beispiel 23

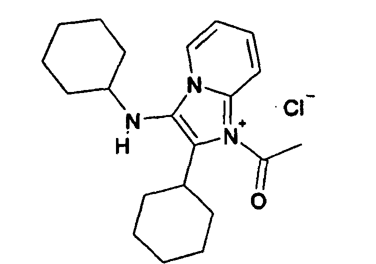

### 1-Acetyl-2-cyclohexyl-3-cyclohexylamino-imidazo[1,2-a]-pyridin-1-iumchlorid

Beispiel **23** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) Cyclohexylisonitril-Lösung (0.2M, DCM), 0.500 ml (0.15 mmol) Cyclohexancarbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 340,49, gefundene Masse M⁺ = 340,5

### Beispiel 24

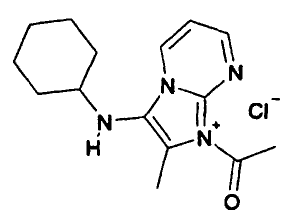

### 1-Acetyl-3-cyclohexylamino-2-methyl-imidazo[1,2-a]-pyrimidin-1-iumchlorid

Beispiel **24** wurde gemäß der allgemeinen Synthesevorschrift im Verfahrensschritt a) aus 1.0 ml (0.1 mmol) 2-Aminopyrimidin (0.1M, DCM), 0.575 ml (0.115 mmol) Cyclohexylisonitril-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 273,63, gefundene Masse M⁺ = 272,3

### Beispiel 25

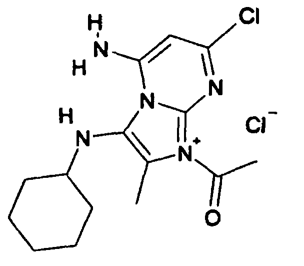

### 1-Acetyl-5-amino-7-chlor-3-cyclohexylamino-2-methylimidazo[1,2-a]pyrimidin-1-iumchlorid

Beispiel **25** wurde gemäß der allgemeinen Synthesevorschrift im Verfahrensschritt a) aus 1.0 ml (0.1 mmol) 2,6-Diamino-4-chlor-pyrimidin (0.1M, DCM), 0.575 ml (0.115 mmol) Cyclohexylisonitril-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 322,82, gefundene Masse M⁺ = 322,3

### Beispiel 26

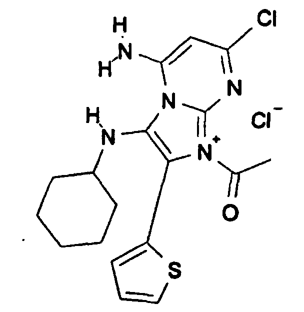

### 1-Acetyl-5-amino-7-chlor-3-cyclohexylamino-2-thiophen-2-yl-imidazo[1,2-a]pyrimidin-1-iumchlorid

Beispiel **26** wurde gemäß der allgemeinen Synthesevorschrift im Verfahrensschritt a) aus 1.0 ml (0.1 mmol) 2,6-Diamino-4-chlor-pyrimidin (0.1M, DCM), 0.575 ml (0.115 mmol) Cyclohexylisonitril-Lösung (0.2M, DCM), 0.500 ml (0.15 mmol) Thiophen-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 322,82, gefundene Masse M⁺ = 322,3

### Beispiel 27

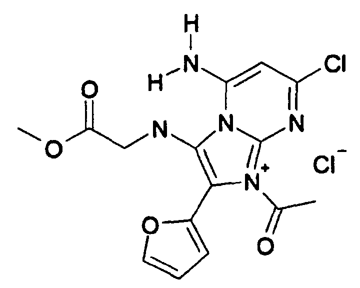

### 1-Acetyl-5-amino-7-chlor-2-furan-2-yl-3-(methoxycarbonylmethylamino)imidazo[1,2-a]pyrimidin-1-iumchlorid

Beispiel **27** wurde gemäß der allgemeinen Synthesevorschrift im Verfahrensschritt a) aus 1.0 ml (0.1 mmol) 2,6-Diamino-4-chlorpyrimidin (0.1M, DCM), 0.575 ml (0.115 mmol) Isocyanessigsäuremethylester-Lösung (0.2M, DCM), 0.500 ml (0.15 mmol) Furfural-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 364,77, gefundene Masse M⁺ = 364,5

### Beispiel 28

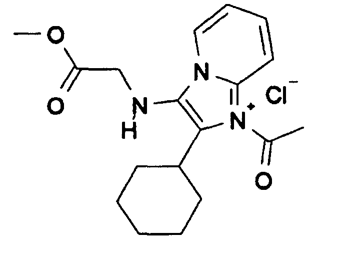

### 1-Acetyl-2-cyclohexyl-3-(methoxycarbonylmethylamino)-imidazo[1,2-a]pyridin-1-iumchlorid

Beispiel **28** wurde gemäß der allgemeinen Synthesevorschrift im Verfahrensschritt a) aus 1.0 ml (0.1 mmol) 2-Aminopyridin (0.1M, DCM), 0.575 ml (0.115 mmol) Isocyanessigsäuremethylester-Lösung (0.2M, DCM), 0.500 ml (0.15 mmol) Cyclohexylcarbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 330,41, gefundene Masse M⁺ = 330,4

### Beispiel 29

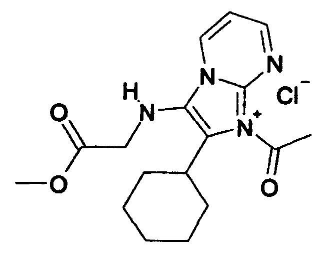

### 1-Acetyl-2-cyclohexyl-3-(methoxycarbonylmethylamino)-imidazo[1,2-a]pyrimidin-1-iumchlorid

Beispiel **29** wurde gemäß der allgemeinen Synthesevorschrift im Verfahrensschritt a) aus 1.0 ml (0.1 mmol) 2-Aminopyrimidin (0.1M, DCM), 0.575 ml (0.115 mmol) Isocyanessigsäuremethylester-Lösung (0.2M, DCM), 0.500 ml (0.15 mmol) Cyclohexylcarbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 331,40 gefundene Masse M⁺ = 331,3

### Beispiel 30

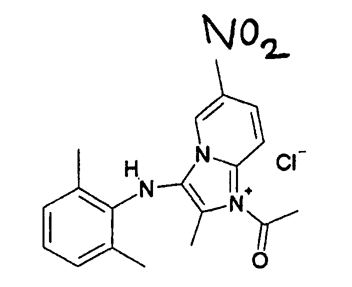

### 1-Acetyl-3-(2,6-dimethyl-phenylamino)-2-methyl-6-nitroimidazo[1,2-a]pyridin-1-iumchlorid

Beispiel **30** wurde gemäß der allgemeinen Synthesevorschrift im Verfahrensschritt a) aus 1.0 ml (0.1 mmol) 2-Amino-5-nitropyridin (0.1M, DCM), 0.575 ml (0.115 mmol) 2,6-Dimethylphenylisocyanid-Lösung (0.2M, DCM), 0.500 ml (0.15 mmol) Acetaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 339,38 gefundene Masse M⁺ = 339,0

### Beispiel 31

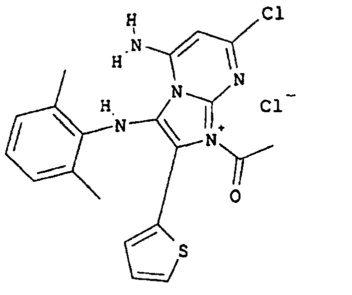

### 1-Acetyl-5-amino-7-chlor-3-(2,6-dimethyl-phenylamino)-2-thiophen-2-ylimidazo[1,2-a]pyrimidin-1-iumchlorid

Beispiel 31 wurde gemäß der allgemeinen Synthesevorschrift im Verfahrensschritt a) aus 1.0 ml (0.1 mmol) 2,6-Diamino-4-chlorpyridin (0.1M, DCM), 0.575 ml (0.115 mmol) 2,6-Dimethylphenylisocyanid-Lösung (0.2M, DCM), 0.500 ml (0.15 mmol) Thiophen-2-carbaldehyd-Lösung (0.3 M, DCM) und 10 µl Perchlorsäure (w = 20%) und im Verfahrensschritt c) und d) durch Umsetzung des erhaltenen Reaktionsprodukts mit 0,4 mmol Acetylchlorid dargestellt.

Charakterisierung durch ESI-MS, berechnete Masse M - Cl⁻ = 412,92 gefundene Masse M⁺ = 412,2

### Beispiel 32

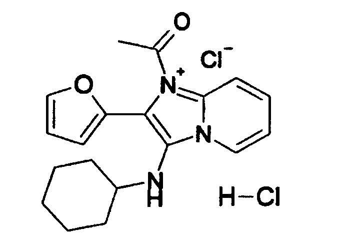

### 1-Acetyl-3-cyclohexylamino-2-furan-2-yl-imidazo[1,2-a]pyridin-1-iumchlorid Hydrochlorid

655 mg (6.1 mmol) 5-Methyl-2-aminopyridin wurde in 12 ml Methanol vorgelegt, anschließend 874 mg (0,75 ml; 9.1 mmol) Furan-2-carbaldehyd, 768 mg (0,86 ml; 7.0 mmol) Cyclohexylisonitril und 0.59 ml wäßrige Perchlorsäure (20 m%) zugegeben und 22 Stunden bei Raumtemperatur gerührt. Zur Aufarbeitung wurden 50 ml Wasser und 40 ml DCM zugegeben, 10 Minuten gerührt und dann die Phasen getrennt. Die wäßrige Phase wurde noch dreimal mit je 20 ml DCM extrahiert und dann die vereinigten organischen Phasen kurz mit 50 ml gesättigter Natriumchloridlösung geschüttelt, abgetrennt, über Magnesiumsulfat getrocknet, filtriert und im Vakuum eingeengt. Das erhaltene Zwischenprodukt (1,83 g; 6,2 mmol) wurde in 10 ml DCM gelöst, und unter Rühren bei 20 °C vier Äquivalente Acetylchlorid (24,8 mmol; 1,8 ml) zugetropft und vier Stunden nachgerührt. Anschließend wurde das Reaktionsgemisch in Vakuum eingeengt und im Ölpumpenvakuum getrocknet. Das Rohprodukt (ca. 2 g) wurde in 15.5 ml 2-Butanon gelöst, 51 µl Wasser und 0.72 ml Trimethylchlorsilan zugegeben und über Nacht gerührt. Das ausgefallene HCl-Addukt von 1-Acetyl-3-cyclohexylamino-2-furan-2-yl-imidazo[1,2-a]pyridin-1-iumchlorid wurde abgesaugt und im Vakuum getrocknet. Es wurden 776 mg 1-Acetyl-3-cyclohexylamino-2-furan-2-yl-imidazo[1,2-a]pyridin-1-iumchlorid Hydrochlorid erhalten.

Charakterisierung durch 300 MHz ¹H-NMR Spektroskopie (keine Verunreinigungen erkennbar).

### Pharmakologische Untersuchungen:

### µ-Opiatrezeptor-Bindungsuntersuchungen

Die Untersuchungen zur Bestimmung der Affinität der erfindungsgemäßen Verbindungen der Formel I zum µ-Opiatrezeptor wurde an Hirnmembranhomogenaten (Homogenat von Rattenhirn ohne Cerebellum, Pons und Medulla oblongata von männlichen Wistar-Ratten) durchgeführt.

Hierzu wurde das jeweils frisch präparierte Rattenhirn unter Eiskühlung in 50 mmol/l Tris-HCl (pH 7,4) homogenisiert und für 10 Minuten bei 5.000 g und 4°C zentrifugiert. Nach Dekantieren und Verwerfen des Überstandes, erneutem Aufnehmen und Homogenisieren des Membransedimentes in 50 mmol/l Tris-HCl (pH 7,4) wurde das Homogenat anschließend für 20 Minuten bei 20.000 g und 4°C zentrifugiert. Dieser Waschschritt wurde nochmals wiederholt. Danach wurde der Überstand dekantiert und das Membransediment in kaltem 50 mmol/l Tris-HCl , 20 % Glycerol (w/v), 0,01 % Bactitracin (w/v) (pH 7,4) homogenisiert und in Aliquoten bis zur Testung eingefroren. Für die Rezeptorbindungstestung wurden die Aliquote aufgetaut und 1:10 mit dem Bindungstest-Puffer verdünnt.

Im Bindungstest wurde als Puffer ein 50 mmol/l Tris-HCL, 5 mmol/l MgCl (pH 7,4), sowie als radioaktiver Ligand 1 nmol/l tritiiertes Naloxon eingesetzt.

| Erfindungsgemäße Verbindung | µ-Affinität % Hemmung 10 µM |
|---|---|
| Beispiel 4 | 90 |
| Beispiel 5 | 79 |
| Beispiel 6 | 53 |
| Beispiel 7 | 91 |
| Beispiel 8 | 98 |

## Patentansprüche

1. Salze von bicyclischen, N-acylierten Imidazo-3-aminen und Imidazo-5-aminen der allgemeinen Formel I, 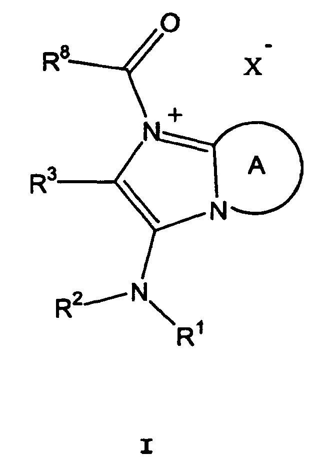 worin
R¹ *tert*-Butyl, 1,1,3,3-Tetramethylbutyl, (CH₂)₆CN, Cyclohexyl, CH₂CO(OMethyl), 2,6-Dimethylphenyl, *tert*-Butyl oder CH₂CH₂R (R = 4-Morpholino), bedeutet,
R² Wasserstoff, COR^{b}, wobei R^{b} für unverzweigtes oder verzweigtes C₁-C₈-Alkyl oder C₃-C₈-Cycloalkyl steht, bedeutet,
R³ ausgewählt ist aus der Gruppe 4-Acetamidophenyl, 2-Bromphenyl, 3-Bromphenyl, 4-Bromphenyl, 4-Brom-2-fluorphenyl, 5-Brom-2-fluorphenyl, 3-Brom-4-fluorphenyl, 4-tert. Butylphenyl, 2-Chlor-4-fluorphenyl, 2-Chlor-6-fluorphenyl, 2-Chlorphenyl, 3-Chlorphenyl, 4-Chlorphenyl, 4-Cyanophenyl, 2,3-Dichlorphenyl, 2,4-Dichlorphenyl, 3,4-Dichlorphenyl, 2,3-Dimethoxyphenyl, 3,4-Dimethoxyphenyl, 2,4-Dimethylphenyl, 2,5-Dimethylphenyl, 2-Fluor-phenyl, 3-Fluorphenyl, 4-Fluorphenyl, 4-Hexylphenyl, 3-Hydroxyphenyl, 2-Methoxyphenyl, 2-Methylphenyl, 3-Methylphenyl, 4-Methylphenyl, 4-Nitrophenyl, 3-Phenoxyphenyl, 4-(1-Pyrrolidino)-phenyl, 2-(Trifluormethyl)phenyl, 3-(Trifluormethyl)phenyl, 4-(Trifluormethyl)-phenyl, 3,4,5-Trimethoxyphenyl, 3-(4-Chlorphenoxy)phenyl, 4-Acetoxy-3-methoxyphenyl, 4-Dimethylaminonaphthyl, 2-Ethoxynaphthyl, 4-Methoxynaphthyl, 2-(1-(Phenylsulfonyl)-pyrrol), 2-(N-Methylpyrrol), 2-(N-(3,5-Dichlorphenyl)pyrrol), 2-(1-(4-Chlorphenyl)pyrrol), 2-(5-Acetoxymethylfurfuryl), 2-(5-Methylfurfuryl), 2-(5-Nitrofurfuryl), 2-[5-(3-Nitrophenyl)furfuryl], 2-[5-(2-Nitrophenyl)furfuryl], 2-(5-Bromfurfuryl), 2-[5-(4-Chlorphenyl)furfuryl], 2-(4,5-Dimethylfurfuryl), 2-[5-(2-Chlorphenyl]furfuryl], 2-(5-Ethylfurfuryl), 2-[5-(1,3-Dioxalanfurfuryl], 2-(5-Chlorthiophenyl), 2-(5-Methylthiophenyl), 2-(5-Ethylthiophenyl), 2-(3-Methylthiophenyl), 2-(4-Bromthiophenyl), 2-(5-Nitrothiophenyl), 5-(2-Carbonsäurethiophenyl), 2-[4-(Phenylethyl)-thiophenyl], 2-[5-(Methylthio)thiophenyl], 2-(3-Bromthiophenyl), 2-(3-Phenoxythiophenyl) oder 2-(5-Bromthiophenyl) oder insbesondere Methyl, Cyclohexyl, Phenyl, Furan-2-yl, 2-Pyridyl oder 2-Thiophenyl,
A ein dreigliedriges Ringfragment ausgewählt aus einer der Formeln 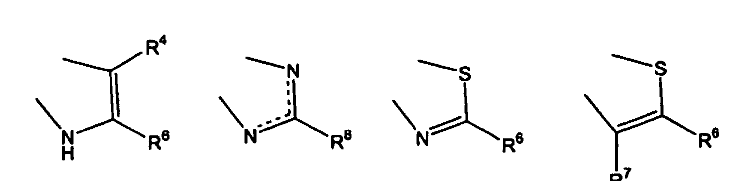 oder ein viergliedriges Ringfragment ausgewählt aus einer der Formeln 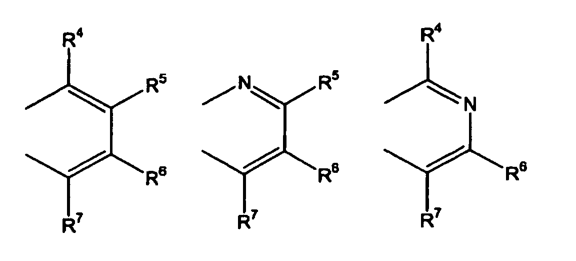 bedeutet, wobei jeweils R⁴, R⁵, R⁶ und R⁷ unabhängig voneinander Wasserstoff, unverzweigtes oder verzweigtes C₁-C₈-Alkyl, Fluor, Chlor, Brom, CF₃, CN, NO₂, NHR^{f}, wobei R^{f} für Wasserstoff, unverzweigtes oder verzweigtes C₁-C₈-Alkyl, oder COR''' (R''' = unverzweigtes oder verzweigtes C₁-C₄-Alkyl oder Phenyl) steht, SR^{g}, wobei R^{g} für Wasserstoff, unverzweigtes oder verzweigtes C₁-C₄-Alkyl, Phenyl, Pyridin, Benzyl oder Fluorenyl steht, OR^{h}, wobei R^{h} für Wasserstoff, unverzweigtes oder verzweigtes C₁-C₈-Alkyl, COR''' (R''' = unverzweigtes oder verzweigtes C₁-C₄-Alkyl oder Phenyl) oder CO(OR') (R' = unverzweigtes oder verzweigtes C₁-C₈-Alkyl) steht, CO(ORⁱ) oder CH₂CO(ORⁱ), wobei Rⁱ jeweils für verzweigtes oder unverzweigtes C₁-C₈-Alkyl oder gegebenenfalls substituiertes Phenyl steht, bedeutet, oder R⁶ und R⁷ gemeinsam ein Ringfragment -CRⁱ=CR^{j}-CH=CHbedeuten, wobei Rⁱ und R^{j} Wasserstoff bedeuten oder einer der beiden Reste Rⁱ oder R^{j} von Wasserstoff verschieden ist und F, Cl, Br, I oder unverzweigtes oder verzweigtes C₁-C₈-Alkyl bedeutet, während R⁴ und R⁵ unabhängig davon die zuvor angegebene Bedeutung haben,
R⁸ verzweigtes oder unverzweigtes C₁-C₈-Alkyl, Aryl, Heteroaryl, C₃-C₈-Cycloalkyl oder einen über eine C₁-C₃-Alkylen-Gruppe gebundenen Aryl, Heteroaryl oder C₃-C₈-Cycloalkylrest bedeutet,
und
X das Anion einer anorganischen oder organischen Säure bedeutet.

2. Salze von bicyclischen, N-acylierten Imidazo-3-aminen und Imidazo-5-aminen gemäß Anspruch 1, **dadurch gekennzeichnet, daß** A ein dreigliedriges Ringfragment der Formel 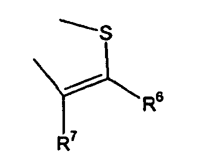 oder ein ein viergliedriges Ringfragment ausgewählt aus einer der Formeln 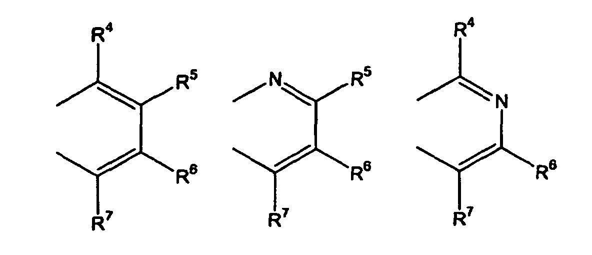 bedeutet.

3. Salze von bicyclischen, N-acylierten Imidazo-3-aminen und Imidazo-5-aminen gemäß einem der Ansprüche 1 oder 2, **dadurch gekennzeichnet, daß** R⁴, R⁵ R⁶ und R⁷ unabhängig voneinander ausgewählt sind aus der Gruppe Wasserstoff, Methyl, Ethyl, Isopropyl, n-Propyl, n-Butyl, Fluor, Brom, CF₃, CN, NO₂, NHR^{f}, wobei R^{f} für Wasserstoff, Methyl, Ethyl, n-Propyl, n-Butyl, C(O)Methyl oder C(O)Phenyl steht, SR^{g}, wobei R^{g} für Wasserstoff, Methyl, Ethyl, n-Propyl, n-Butyl oder Phenyl steht, OR^{h}, wobei R^{h} für Wasserstoff, Methyl, Ethyl, n-Propyl, n-Butyl, Phenyl, COMethyl, COPhenyl CO(OMethyl) oder CO(OEthyl) steht, CO(ORⁱ) oder CH₂CO(ORⁱ), wobei Rⁱ jeweils für Methyl, Ethyl, n-Propyl, n-Butyl oder Phenyl steht, wobei Wasserstoff, Methyl, Chlor, NH₂, und NO₂ besonders bevorzugt sind.

4. Salze von bicyclischen, N-acylierten Imidazo-3-aminen und Imidazo-5-aminen gemäß einem der Ansprüche 1, 2 oder 3, **dadurch gekennzeichnet, daß** R⁸ Methyl, Ethyl, n-Propyl, n-Butyl, n-Pentyl, n-Hexyl, unsubstituiertes, in 4-Position monosubstituiertes oder in 2- und 6-Position disubstituiertes Phenyl, Cyclohexyl oder 2-Naphtyl bedeutet, wobei Methyl, n-Hexyl, 2,6-Dichlorphenyl, 4-Methoxyphenyl, Cyclohexyl oder 2-Naphtyl besonders bevorzugt sind.

5. Salze von bicyclischen, N-acylierten Imidazo-3-aminen und Imidazo-5-aminen gemäß einem der Ansprüche 1, 2, 3 oder 4, **dadurch gekennzeichnet, daß** X für das Anion der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure oder Asparaginsäure oder insbesondere der Salzsäure steht.

6. Salze von bicyclischen, N-acylierten Imidazo-3-aminen und Imidazo-5-aminen gemäß einem der Ansprüche 1, 2, 3, 4 oder 5 in Form ihrer Additionsprodukte mit Säuren, vorzugsweise mit physiologisch verträglichen Säuren.

7. Salze von bicyclischen, N-acylierten Imidazo-3-aminen und Imidazo-5-aminen gemäß einem der Ansprüche 1, 2, 3, 4 oder 5 oder deren Additionsprodukte mit Säuren gemäß Anspruch 6, **dadurch gekennzeichnet, daß** diese ausgewählt sind aus der Gruppe
1-Acetyl-3-*tert*-butylamino-2-phenyl-imidazo[1,2-a]-pyridin-1-iumchlorid
1-Acetyl-3-*tert*-butylamino-2-phenyl-imidazo[1,2-a]-pyrazin-1-iumchlorid
1-Acetyl-3-*tert*-butylamino-2-phenyl-imidazo[1,2-a]-pyrimidin-1-iumchlorid
7-Acetyl-5-*tert*-butylamino-6-phenyl-imidazo[2,1-b]-thiazol-7-iumchlorid
3-Acetyl-1-cyclohexylamino-2-phenyl-imidazo[1,2-a]-chinolin-3-iumchlorid
1-Acetyl-3-cyclohexylamino-2-phenyl-imidazo[1,2-a]-pyrimidin-1-iumchlorid
1-Acetyl-8-benzyloxy-3-cyclohexylamino-2-methylimidazo[1,2-a]pyridin-1-iumchlorid
1-Acetyl-8-benzyloxy-3-*tert*-butylamino-2-methylimidazo[1,2-a]pyridin-1-iumchlorid
1-Acetyl-8-benzyloxy-2-methyl-3-(1,1,3,3-tetramethylbutylamino)imidazo[1,2-a]pyridin-1-iumchlorid
3-(*tert*-Butyl-cyclohexancarbonyl-amino)-1-cyclohexancarbonyl-2-phenyl-imidazo[1,2-a]pyridin-1-iumchlorid
3-(*tert*-Butyl-heptanoyl-amino)-1-heptanoyl-2-phenyl-imidazo[1,2-a]pyridin-1-iumchlorid
1-Acetyl-3-cyclohexylamino-2-furan-2-ylimidazo[1,2-a]pyridin-1-iumchlorid
1-Acetyl-3-cyclohexylamino-2-furan-2-ylimidazo[1,2-a]pyrimidin-1-iumchlorid
1-Acetyl-5-amino-7-chlor-3-cyclohexylamino-2-furan-2-ylimidazo[1,2-a]pyrimidin-1-iumchlorid
1-Acetyl-3-cyclohexylamino-2-pyridin-2-yl-imidazo-[1,2-a]pyridin-1-iumchlorid
1-Acetyl-3-cyclohexylamino-6-nitro-2-pyridin-2-ylimidazo[1,2-a]pyridin-1-iumchlorid
1-Acetyl-3-cyclohexylamino-5-methyl-2-pyridin-2-ylimidazo[1,2-a]pyridin-1-iumchlorid
1-Acetyl-3-cyclohexylamino-2-pyridin-2-ylimidazo[1,2-a]pyrazin-1-iumchlorid
1-Acetyl-3-cyclohexylamino-2-pyridin-2-yl-imidazo-[1,2-a]pyrimidin-1-iumchlorid
1-Acetyl-5-amino-7-chlor-3-cyclohexylamino-2-pyridin-2-ylimidazo[1,2-a]pyrimidin-1-iumchlorid
1-Acetyl-3-cyclohexylamino-5,7-dimethyl-2-pyridin-2-yl-imidazo[1,2-a]pyrimidin-1-iumchlorid
7-Acetyl-5-cyclohexylamino-6-pyridin-2-yl-imidazo-[2,1-b]thiazol-7-iumchlorid
1-Acetyl-2-cyclohexyl-3-cyclohexylaminoimidazo[1,2-a]pyridin-1-iumchlorid
1-Acetyl-3-cyclohexylamino-2-methyl-imidazo[1,2-a]-pyrimidin-1-iumchlorid
1-Acetyl-5-amino-7-chlor-3-cyclohexylamino-2-methyl-imidazo[1,2-a]pyrimidin-1-iumchlorid
1-Acetyl-5-amino-7-chlor-3-cyclohexylamino-2-thiophen-2-yl-imidazo[1,2-a]pyrimidin-1-iumchlorid
1-Acetyl-5-amino-7-chlor-2-furan-2-yl-3-(methoxycarbonylmethylamino)imidazo[1,2-a]pyrimidin-1-iumchlorid
1-Acetyl-2-cyclohexyl-3-(methoxycarbonylmethylamino)-imidazo[1,2-a]pyridin-1-iumchlorid
1-Acetyl-2-cyclohexyl-3-(methoxycarbonylmethylamino)-imidazo[1,2-a]pyrimidin-1-iumchlorid
1-Acetyl-3-(2,6-dimethyl-phenylamino)-2-methyl-6-nitro-imidazo[1,2-a]pyridin-1-iumchlorid
1-Acetyl-5-amino-7-chlor-3-(2,6-dimethyl-phenylamino)-2-thiophen-2-ylimidazo[1,2-a]pyrimidin-1-iumchlorid
1-Acetyl-3-cyclohexylamino-2-furan-2-ylimidazo[1,2-a]pyridin-1-iumchlorid Hydrochlorid

8. Verfahren zur Herstellung der Salze bicyclischer, N-acylierten Imidazo-3-amine und Imidazo-5-amine und ihrer Additionsprodukte mit Säuren gemäß einem der Ansprüche 1 bis 7 umfassend die Schritte
a) Herstellung der Imidazo-3-amine bzw. Imidazo-5-amine durch Dreikomponentenreaktion aus Amidin, Aldehyd und Isonitril in einem Lösungsmittel, vorzugsweise Dichlormethan, und in Gegenwart einer Säure, vorzugsweise Perchlorsäure, wobei die Ausgangsverbindungen nacheinander in der Reihenfolge Amidin, Aldehyd und Isonitril zugegeben werden,
b) gegebenenfalls zur Herstellung von Verbindungen, bei denen R² nicht Wasserstoff bedeutet, Umsetzung der in Schritt a) entstehenden Produkte mit einer Verbindung R²Hal (Hal = Brom, Iod oder insbesondere Chlor) oder einem Isocyanat,
c) Umsetzung des Reaktionsprodukts aus Schritt a) bzw. b) mit einem vorzugsweise mindestens vierfachen molaren Überschuß eines Säurechlorids R⁸C(O)Cl,
d) Nach Reaktionsende Entfernung des überschüssigen Säurechlorids aus dem Reaktionsgemisch,
e) Gegebenenfalls in an sich bekannter Weise Austausch des Chlorids gegen einen anderen Säurerest vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure oder Asparaginsäure.
f) Gegebenenfalls Herstellung des Additionsprodukts mit einer Säure, vorzugsweise einer physiologisch verträglichen Säure.

9. Verfahren gemäß Anspruch 8, **dadurch gekennzeichnet, daß** Verfahrenschritt c) in einem Ether oder Halogenkohlenwasserstoff, vorzugsweise THF oder DCM als Lösungsmittel bei einer Temperatur von 0-60°C durchgeführt wird.

10. Verfahren gemäß Anspruch 8 oder 9, **dadurch gekennzeichnet, daß** Verfahrenschritt d) 2-12 Stunden nach Zugabe des Säurechlorids in Verfahrenschritt c) erfolgt.

11. Verfahren gemäß einem der Ansprüche 8 bis 9, **dadurch gekennzeichnet, daß** in Verfahrenschritt d) die Entfernung des überschüssigen Säurechlorids Mittels eines Scavenger-Harzes, insbesondere mittels (Tris-(2-Aminoethyl)amin-polystyrol erfolgt.

12. Arzneimittel enthaltend als Wirkstoff mindestens ein Salz eines bicyclischen, N-acylierten Imidazo-3-amins oder Imidazo-5-amins der allgemeinen Formel I gemäß Anspruch 1 und/oder mindestens ein Additionsprodukt mit einer physiologisch verträglichen Säure gemäß Anspruch 6, in der R¹ bis R⁸, A und X die in Anspruch 1 angegebene Bedeutung haben.

13. Arzneimittel gemäß Anspruch 12, **dadurch gekennzeichnet, daß** es als Wirkstoff mindestens ein ein Salz eines bicyclischen, N-acylierten Imidazo-3-amins oder Imidazo-5-amins oder dessen Säureadditionsprodukt enthält ausgewählt aus der Gruppe
1-Acetyl-3-*tert*-butylamino-2-phenyl-imidazo[1,2-a]-pyridin-1-iumchlorid
1-Acetyl-3-*tert*-butylamino-2-phenyl-imidazo[1,2-a]-pyrazin-1-iumchlorid
1-Acetyl-3-*tert*-butylamino-2-phenyl-imidazo[1,2-a]-pyrimidin-1-iumchlorid
7-Acetyl-5-*tert*-butylamino-6-phenyl-imidazo[2,1-b]-thiazol-7-iumchlorid
3-Acetyl-1-cyclohexylamino-2-phenyl-imidazo[1,2-a]-chinolin-3-iumchlorid
1-Acetyl-3-cyclohexylamino-2-phenyl-imidazo[1,2-a]-pyrimidin-1-iumchlorid
1-Acetyl-8-benzyloxy-3-cyclohexylamino-2-methylimidazo[1,2-a]pyridin-1-iumchlorid
1-Acetyl-8-benzyloxy-3-*tert*-butylamino-2-methylimidazo[1,2-a]pyridin-1-iumchlorid
1-Acetyl-8-benzyloxy-2-methyl-3-(1,1,3,3-tetramethylbutylamino)imidazo[1,2-a]pyridin-1-iumchlorid
3-(*tert*-Butyl-cyclohexancarbonyl-amino)-1-cyclohexancarbonyl-2-phenyl-imidazo[1,2-a]pyridin-1-iumchlorid
3-(*tert*-Butyl-heptanoyl-amino)-1-heptanoyl-2-phenylimidazo[1,2-a]pyridin-1-iumchlorid
1-Acetyl-3-cyclohexylamino-2-furan-2-yl-imidazo[1,2-a]pyridin-1-iumchlorid
1-Acetyl-3-cyclohexylamino-2-furan-2-yl-imidazo[1,2-a]pyrimidin-1-iumchlorid
1-Acetyl-5-amino-7-chlor-3-cyclohexylamino-2-furan-2-ylimidazo[1,2-a]pyrimidin-1-iumchlorid
1-Acetyl-3-cyclohexylamino-2-pyridin-2-yl-imidazo-[1,2-a]pyridin-1-iumchlorid
1-Acetyl-3-cyclohexylamino-6-nitro-2-pyridin-2-ylimidazo[1,2-a]pyridin-1-iumchlorid
1-Acetyl-3-cyclohexylamino-5-methyl-2-pyridin-2-ylimidazo[1,2-a]pyridin-1-iumchlorid
1-Acetyl-3-cyclohexylamino-2-pyridin-2-ylimidazo[1,2-a]pyrazin-1-iumchlorid
1-Acetyl-3-cyclohexylamino-2-pyridin-2-yl-imidazo-[1,2-a]pyrimidin-1-iumchlorid
1-Acetyl-5-amino-7-chlor-3-cyclohexylamino-2-pyridin-2-ylimidazo[1,2-a]pyrimidin-1-iumchlorid
1-Acetyl-3-cyclohexylamino-5,7-dimethyl-2-pyridin-2-yl-imidazo[1,2-a]pyrimidin-1-iumchlorid
7-Acetyl-5-cyclohexylamino-6-pyridin-2-yl-imidazo-[2,1-b]thiazol-7-iumchlorid
1-Acetyl-2-cyclohexyl-3-cyclohexylaminoimidazo[1,2-a]pyridin-1-iumchlorid
1-Acetyl-3-cyclohexylamino-2-methyl-imidazo[1,2-a]-pyrimidin-1-iumchlorid
1-Acetyl-5-amino-7-chlor-3-cyclohexylamino-2-methyl-imidazo[1,2-a]pyrimidin-1-iumchlorid
1-Acetyl-5-amino-7-chlor-3-cyclohexylamino-2-thiophen-2-yl-imidazo[1,2-a]pyrimidin-1-iumchlorid
1-Acetyl-5-amino-7-chlor-2-furan-2-yl-3-(methoxycarbonylmethylamino)imidazo[1,2-a]pyrimidin-1-iumchlorid
1-Acetyl-2-cyclohexyl-3-(methoxycarbonylmethylamino)-imidazo[1,2-a]pyridin-1-iumchlorid
1-Acetyl-2-cyclohexyl-3-(methoxycarbonylmethylamino)-imidazo[1,2-a]pyrimtidin-1-iumchlorid
1-Acetyl-3-(2,6-dimethyl-phenylamino)-2-methyl-6-nitro-imidazo[1,2-a]pyridin-1-iumchlorid
1-Acetyl-5-amino-7-chlor-3-(2,6-dimethylphenylamino)-2-thiophen-2-ylimidazo[1,2-a]pyrimidin-1-iumchlorid
1-Acetyl-3-cyclohexylamino-2-furan-2-ylimidazo[1,2-a]pyridin-1-iumchlorid Hydrochlorid.

14. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Schmerz.

15. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Drogen- und/oder Alkoholmißbrauch.

16. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Diarrhoe.

17. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Gastritis.

18. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Ulcera.

19. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Harninkontinenz.

20. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Depressionen.

21. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Narkolepsie.

22. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Übergewicht.

23. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Asthma.

24. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Glaukom.

25. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Tinnitus.

26. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von Juckreiz.

27. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung des hyperkinetischen Syndroms.

28. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von oder Prophylaxe bei Epilepsie.

29. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Behandlung von oder Prophylaxe bei Schizophrenie.

30. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels zur Anxiolyse.

31. Verwendung von Salzen eines bicyclischen, N-acylierten Imidazo-3-amins oder Imidazo-5-amins der allgemeinen Formel I gemäß Anspruch 1 und/oder deren Additionsprodukten mit physiologisch verträglichen Säuren gemäß Anspruch 6, in denen R¹ bis R⁸, A und X die in Anspruch 1 angegebene Bedeutung haben, zur Herstellung eines Arzneimittels mit anästhestischer Wirkung.

## Claims

1. Salts of bicyclic, N-acylated imidazo-3-amines and imidazo-5-amines of general Formula I 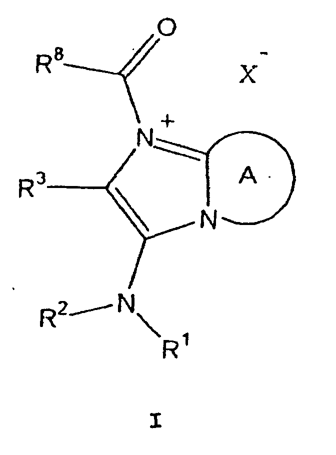 wherein
R¹ represents *tert*-butyl, 1,1,3,3-tetramethylbutyl, (CH₂)₆CN, cyclohexyl, CH₂CO(Omethyl), 2,6-dimethylphenyl, *tert*-butyl or CH₂CH₂R (R = 4-morpholino),
R² represents hydrogen, COR^{b}, wherein R^{b} represents straight or branched C₁-C₈ alkyl or C₃-C₈ cycloalkyl,
R³ is selected from the group comprising 4-acetamidophenyl, 2-bromophenyl, 3-bromophenyl, 4-bromophenyl, 4-bromo-2-fluorophenyl, 5-bromo-2-fluorophenyl, 3-bromo-4-fluorophenyl, 4-tert. butylphenyl, 2-chloro-4-fluorophenyl, 2-chloro-6-fluorophenyl, 2-chlorophenyl, 3-chlorophenyl, 4-chlorophenyl, 4-cyanophenyl, 2,3-dichlorophenyl, 2,4-dichlorophenyl, 3,4-dichlorophenyl, 2,3-dimethoxyphenyl, 3,4-dimethoxyphenyl, 2,4-dimethylphenyl, 2,5-dimethylphenyl, 2-fluorophenyl, 3-fluorophenyl, 4-fluorophenyl, 4-hexylphenyl, 3-hydroxyphenyl, 2-methoxyphenyl, 2-methylphenyl, 3-methylphenyl, 4-methylphenyl, 4-nitrophenyl, 3-phenoxyphenyl, 4-(1-pyrrolidino)-phenyl, 2-(trifluoromethyl)phenyl, 3-(trifluoromethyl)phenyl, 4-(trifluoromethyl)-phenyl, 3,4,5-trimethoxyphenyl, 3-(4-chlorophenoxy)phenyl, 4-acetoxy-3-methoxyphenyl, 4-dimethylaminonaphthyl, 2-ethoxynaphthyl, 4-methoxynaphthyl, 2-(1-(phenylsulphonyl)-pyrrole), 2-(N-methylpyrrole), 2-(N-(3,5-dichlorophenyl)pyrrole), 2-(1-(4-chlorophenyl)pyrrole), 2-(5-acetoxymethylfurfuryl), 2-(5-methylfurfuryl), 2-(5-nitrofurfuryl), 2-[5-(3-nitrophenyl)furfuryl], 2-[5-(2-nitrophenyl)furfuryl], 2-(5-bromofurfuryl), 2-[5-(4-chlorophenyl)furfuryl], 2-(4,5-dimethylfurfuryl),2-[5-(2-chlorophenyl)furfuryl], 2-(5-ethylfurfuryl), 2-[5-(1,3-dioxalanfurfuryl], 2-(5-chlorothiophenyl), 2-(5-methylthiophenyl), 2-(5-ethylthiophenyl), 2-(3-methylthiophenyl), 2-(4-bromothiophenyl), 2-(5-nitrothiophenyl), 5-(2-carboxylic acid thiophenyl), 2-[4-(phenylethyl)-thiophenyl], 2-[5-(methylthio)thiophenyl], 2-(3-bromothiophenyl), 2-(3-phenoxythiophenyl) or 2-(5-bromothiophenyl) or, in particular, methyl, cyclohexyl, phenyl, furan-2-yl, 2-pyridyl or 2-thiophenyl,
A represents a three-membered ring fragment selected from one of the formulae 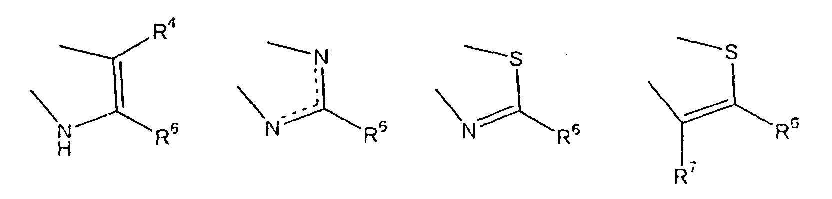 or a four-membered ring fragment selected from one of the formulae 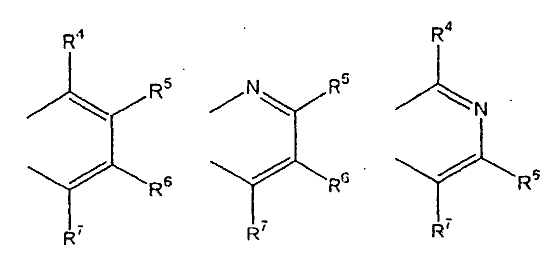 wherein R⁴, R⁵, R⁶ and R⁷ each independently represent hydrogen, straight or branched C₁-C₈ alkyl, fluorine, chlorine, bromine, CF₃, CN, NO₂, NHR^{f}, wherein R^{f} represents hydrogen, straight or branched C₁-C₈ alkyl, or COR''' (R''' = straight or branched C₁-C₄ alkyl or phenyl), SR^{g}, wherein R^{g} represents hydrogen, straight or branched C₁-C₄ alkyl, phenyl, pyridine, benzyl or fluorenyl, OR^{h}, wherein R^{h} represents hydrogen, straight or branched C₁-C₈ alkyl, COR''' (R''' = straight or branched C₁-C₄ alkyl or phenyl) or CO(OR') (R' = straight or branched C₁-C₈ alkyl), CO(ORⁱ) or CH₂CO(ORⁱ), wherein Rⁱ respectively represents branched or straight C₁-C₈ alkyl or optionally substituted phenyl, or R⁶ and R⁷ together represent a ring fragment -CRⁱ=CR^{j}-CH=CH-, wherein Rⁱ and R^{j} represent hydrogen or one of the two radicals Rⁱ or R^{j} differs from hydrogen and F, Cl, Br, I or straight or branched C₁-C₈ alkyl, whereas R⁴ and R⁵ independently thereof have the meaning given above,
R⁸ represents branched or straight C₁-C₈ alkyl, aryl, heteroaryl, C₃-C₈ cycloalkyl or an aryl bound by a C₁-C₃ alkylene group, heteroaryl or C₃-C₈ cycloalkyl radical, and
X represents the anion of an inorganic or organic acid.

2. Salts of bicyclic, N-acylated imidazo-3-amines and imidazo-5-amines according to claim 1, **characterised in that** A represents a three-membered ring fragment of formula 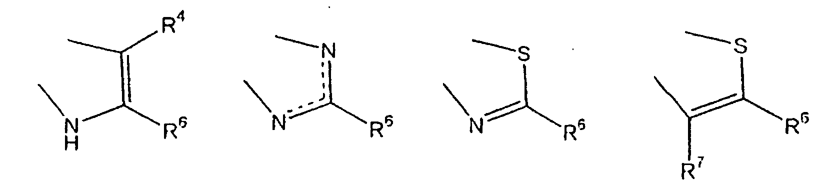 or a four-membered ring fragment selected from one of the formulae

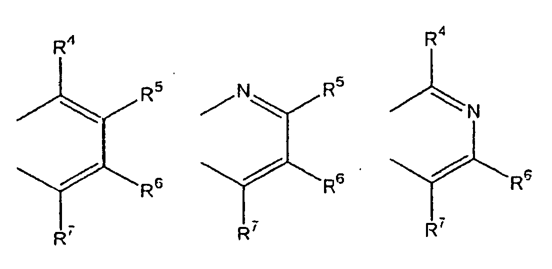

3. Salts of bicyclic, N-acylated imidazo-3-amines and imidazo-5-amines according to one of claims 1 or 2, **characterised in that** R⁴, R⁵, R⁶ and R⁷ are selected independently from one another from the group comprising hydrogen, methyl, ethyl, isopropyl, n-propyl, n-butyl, fluorine, bromine, CF₃, CN, NO₂, NHR^{f}, wherein R^{f} represents hydrogen, methyl, ethyl, n-propyl, n-butyl, C(O)methyl or C(O)phenyl, SR^{g}, wherein R^{g}· represents hydrogen, methyl, ethyl, n-propyl, n-butyl or phenyl, OR^{h}, wherein R^{h} represents hydrogen, methyl, ethyl, n-propyl, n-butyl, phenyl, COmethyl, COphenyl CO(Omethyl) or CO(Oethyl), CO(ORⁱ) or CH₂CO(OR^{j}), wherein Rⁱ respectively represents methyl, ethyl, n-propyl, n-butyl or phenyl, wherein hydrogen, methyl, chlorine, NH₂ and NO₂ are particularly preferred.

4. Salts of bicyclic, N-acylated imidazo-3-amines and imidazo-5-amines according to one of claims 1, 2 or 3, **characterised in that** R⁸ represents methyl, ethyl, n-propyl, n-butyl, n-pentyl, n-hexyl, or phenyl, cycolhexyl or 2-naphthyl unsubstituted, monosubstituted in the 4-position or disubstituted in the 2- and 6-position, wherein methyl, n-hexyl, 2,6-dichlorophenyl, 4-methoxyphenyl, cyclohexyl or 2-naphthyl are particularly preferred.

5. Salts of bicyclic, N-acylated imidazo-3-amines and imidazo-5-amines according to one of claims 1, 2, 3 or 4, **characterised in that** X represents the anion of hydrobromic acid, sulphuric acid, methane sulphonic acid, formic acid, acetic acid, oxalic acid, succinic acid, tartaric acid, mandelic acid, fumaric acid, lactic acid, citric acid, glutamic acid or aspartic acid or, in particular, hydrochloric acid.

6. Salts of bicyclic, N-acylated imidazo-3-amines and imidazo-5-amines according to one of claims 1, 2, 3, 4 or 5 in the form of their addition products with acids, preferably with physiologically acceptable acids.

7. Salts of bicyclic, N-acylated imidazo-3-amines and imidazo-5-amines according to one of claims 1, 2, 3, 4 or 5 or their addition products with acids according to claim 6, **characterised in that** they are selected from the group comprising
1-acetyl-3-*tert*-butylamino-2-phenyl-imidazo[1,2-a]-pyridin-1-iumchloride
1-acetyl-3-*tert*-butylamino-2-phenyl-imidazo[1,2-a]-pyrazin-1-iumchloride
1-acetyl-3-*tert*-butylamino-2-phenyl-imidazo[1,2-a]-pyrimidin-1-iumchloride
7-acetyl-5-tert-butylamino-6-phenyl-imidazo[2,1-b]-thiazol-7-iumchloride
3-acetyl-1-cyclohexylamino-2-phenyl-imidazo[1,2-a]-quinolin-3-iumchloride
1-acetyl-3-cyclohexylamino-2-phenyl-imidazo[1,2-a]-pyrimidin-1-iumchloride
1-acetyl-8-benzyloxy-3-cyclohexylamino-2-methylimidazo[1,2-a]pyridin-1-iumchloride
1-acetyl-8-benzyloxy-3-*tert*-butylamino-2-methylimidazo[1,2-a]pyridin-1-iumchloride
1-acetyl-8-benzyloxy-2-methyl-3-(1,1,3,3-tetramethylbutylamino)imidazo[1,2-a]pyridin-1-iumchloride
3-(*tert*-butyl-cyclohexancarbonyl-amino)-1-cyclohexancarbonyl-2-phenyl-imidazo[1,2-a]pyridin-1-iumchloride
3-(*tert*-butyl-heptanoyl-amino)-1-heptanoyl-2-phenylimidazo[1,2-a]pyridin-1-iumchloride
1-acetyl-3-cyclohexylamino-2-furan-2-yl-imidazo[1,2-a]pyridin-1-iumchloride
1-acetyl-3-cyclohexylamino-2-furan-2-yl-imidazo[1,2-a]pyrimidin-1-iumchloride
1-acetyl-5-amino-7-chloro-3-cyclohexylamino-2-furan-2-yl-imidazo[1,2-a]pyrimidin-1-iumchloride
1-acetyl-3-cyclohexylamino-2-pyridin-2-yl-imidazo[1,2-a]pyridin-1-iumchloride
1-acetyl-3-cyclohexylamino-6-nitro-2-pyridin-2-yl-imidazo[1,2-a]pyridin-1-iumchloride
1-acetyl-3-cyclohexylamino-5-methyl-2-pyridin-2-yl-imidazo[1,2-a]pyridin-1-iumchloride
1-acetyl-3-cyclohexylamino-2-pyridin-2-yl-imidazo[1,2-a]pyrazin-1-iumchloride
1-acetyl-3-cyclohexylamino-2-pyridin-2-yl-imidazo[1,2-a]pyrimidin-1-iumchloride
1-acetyl-5-amino-7-chloro-3-cyclohexylamino-2-pyridin-2-yl-imidazo[1,2-a]pyrimidin-1-iumchloride
1-acetyl-3-cyclohexylamino-5,7-dimethyl-2-pyridin-2-yl-imidazo[1,2-a]pyrimidin-1-iumchloride
7-acetyl-5-cyclohexylamino-6-pyridin-2-yl-imidazo[2,1-b]thiazol-7-iumchloride
1-acetyl-2-cyclohexyl-3-cyclohexylamino-imidazo[1,2-a]pyridin-1-iumchloride
1-acetyl-3-cyclohexylamino-2-methyl-imidazo[1,2-a]pyrimidin-1-iumchloride
1-acetyl-5-amino-7-chloro-3-cyclohexylamino-2-methylimidazo[1,2-a]pyrimidin-1-iumchloride
1-acetyl-5-amino-7-chloro-3-cyclohexylamino-2-thiophene-2-yl-imidazo[1,2-a]pyrimidin-1-iumchloride
1-acetyl-5-amino-7-chloro-2-furan-2-yl-3-(methoxycarbonylmethylamino)imidazo[1,2-a]pyrimidin-1-iumchloride
1-acetyl-2-cyclohexyl-3-(methoxycarbonylmethylamino)imidazo[1,2-a]pyridin-1-iumchloride
1-acetyl-2-cyclohexyl-3-(methoxycarbonylmethylamino)imidazo[1,2-a]pyrimidin-1-iumchloride
1-acetyl-3-(2,6-dimethyl-phenylamino)-2-methyl-6-nitro-imidazo[1,2-a]pyridin-1-iumchloride
1-acetyl-5-amino-7-chloro-3-(2,6-dimethyl-phenylamino)-2-thiophene-2-yl-imidazo[1,2-a]pyrimidin-1-iumchloride
1-acetyl-3-cyclohexylamino-2-furan-2-yl-imidazo[1,2-a]pyridin-1-iumchloride hydrochloride.

8. Process for producing the salts of bicyclic, N-acylated imidazo-3-amines and imidazo-5-amines and their addition products with acids according to one of claims 1 to 7 comprising the steps of
a) producing the imidazo-3-amines and imidazo-5-amines by three-component reaction from amidine, aldehyde and isonitrile in a solvent, preferably dichloromethane, and in the presence of an acid, preferably perchloric acid, wherein the starting compounds are added in succession in the sequence amidine, aldehyde and isonitrile,
b) optionally for producing compounds in which R² is not hydrogen, reacting the products formed in step a) with a compound R²Hal (Hal = bromine, iodine or in particular chlorine) or an isocyanate,
c) converting the reaction product from step a) or b) with a preferably at least quadruple molar excess of an acid chloride R⁸C(O)Cl,
d) removing the excess acid chloride from the reaction mixture on completion of the reaction,
e) optionally exchanging the chloride in a manner known *per se* for a different acid radical, preferably of hydrobromic acid, sulphuric acid, methane sulphonic acid, formic acid, acetic acid, oxalic acid, succinic acid, tartaric acid, mandelic acid, fumaric acid, lactic acid, citric acid, glutamic acid or aspartic acid,
f) optionally producing the addition product with an acid, preferably a physiologically acceptable acid.

9. Process according to claim 8, **characterised in that** process step c) is carried out in an ether or halogenated hydrocarbon, preferably THF or DCM as solvent at a temperature of 0 to 60°C.

10. Process according to either claim 8 or claim 9, **characterised in that** process step d) takes place 2 to 12 hours after addition of the acid chloride in process step c).

11. Process according to one of claims 8 to 9, **characterised in that** the excess acid chloride is removed using a scavenger resin, in particular using (tris-(2-aminoethyl)amine polystyrene in process step d).

12. Pharmaceutical composition containing, as active ingredient, at least one salt of bicyclic, N-acylated imidazo-3-amine and imidazo-5-amine of general Formula I according to claim 1 and/or at least one addition product with a physiologically acceptable acid according to claim 6, in which R¹ to R⁸, A and X have the meaning given in claim 1.

13. Pharmaceutical composition according to claim 12, **characterised in that** it contains, as active ingredient, at least one salt of a bicyclic, N-acylated imidazo-3-amine or imidazo-5-amine or its acid addition product selected from the group comprising
1-acetyl-3-*tert*-butylamino-2-phenyl-imidazo[1,2-a]-pyridin-1-iumchloride
1-acetyl-3-*tert*-butylamino-2-phenyl-imidazo[1,2-a]-pyrazin-1-iumchloride
1-acetyl-3-*tert*-butylamino-2-phenyl-imidazo[1,2-a]-pyrimidin-1-iumchloride
7-acetyl-5-*tert*-butylamino-6-phenyl-imidazo[2,1-b]-thiazol-7-iumchloride
3-acetyl-1-cyclohexylamino-2-phenyl-imidazo[1,2-a]-quinolin-3-iumchloride
1-acetyl-3-cyclohexylamino-2-phenyl-imidazo[1,2-a]-pyrimidin-1-iumchloride
1-acetyl-8-benzyloxy-3-cyclohexylamino-2-methyl-imidazo[1,2-a]pyridin-1-iumchloride
1-acetyl-8-benzyloxy-3-*tert*-butylamino-2-methyl-imidazo[1,2-a]pyridin-1-iumchloride
1-acetyl-8-benzyloxy-2-methyl-3-(1,1,3,3-tetramethylbutylamino)imidazo[1,2-a]pyridin-1-iumchloride
3-(*tert*-butyl-cyclohexancarbonyl-amino)-1-cyclohexancarbonyl-2-phenyl-imidazo[1,2-a]pyridin-1-iumchloride
3-(*tert*-butyl-heptanoyl-amino)-1-heptanoyl-2-phenyl-imidazo[1,2-a]pyridin-1-iumchloride
1-acetyl-3-cyclohexylamino-2-furan-2-yl-imidazo[1,2-a]pyridin-1-iumchloride
1-acetyl-3-cyclohexylamino-2-furan-2-yl-imidazo[1,2-a]pyrimidin-1-iumchloride
1-acetyl-5-amino-7-chloro-3-cyclohexylamino-2-furan-2-yl-imidazo[1,2-a]pyrimidin-1-iumchloride
1-acetyl-3-cyclohexylamino-2-pyridin-2-yl-imidazo[1,2-a]pyridin-1-iumchloride
1-acetyl-3-cyclohexylamino-6-nitro-2-pyridin-2-yl-imidazo[1,2-a]pyridin-1-iumchloride
1-acetyl-3-cyclohexylamino-5-methyl-2-pyridin-2-yl-imidazo[1,2-a]pyridin-1-iumchloride
1-acetyl-3-cyclohexylamino-2-pyridin-2-yl-imidazo[1,2-a]pyrazin-1-iumchloride
1-acetyl-3-cyclohexylamino-2-pyridin-2-yl-imidazo[1,2-a]pyrimidin-1-iumchloride
1-acetyl-5-amino-7-chloro-3-cyclohexylamino-2-pyridin-2-yl-imidazo[1,2-a]pyrimidin-1-iumchloride
1-acetyl-3-cyclohexylamino-5,7-dimethyl-2-pyridin-2-yl-imidazo[1,2-a]pyrimidin-1-iumchloride
7-acetyl-5-cyclohexylamino-6-pyridin-2-yl-imidazo[2,1-b]thiazol-7-iumchloride
1-acetyl-2-cyclohexyl-3-cyclohexylamino-imidazo[1,2-a]pyridin-1-iumchloride
1-acetyl-3-cyclohexylamino-2-methyl-imidazo[1,2-a]pyrimidin-1-iumchloride
1-acetyl-5-amino-7-chloro-3-cyclohexylamino-2-methyl-imidazo[1,2-a]pyrimidin-1-iumchloride
1-acetyl-5-amino-7-chloro-3-cyclohexylamino-2-thiophene-2-yl-imidazo[1,2-a]pyrimidin-1-iumchloride
1-acetyl-5-amino-7-chloro-2-furan-2-yl-3-(methoxycarbonylmethylamino)imidazo[1,2-a]pyrimidin-1-iumchloride
1-acetyl-2-cyclohexyl-3-(methoxycarbonylmethylamino)imidazo[1,2-a]pyridin-1-iumchloride
1-acetyl-2-cyclohexyl-3-(methoxycarbonylmethylamino)imidazo[1,2-a]pyrimidin-1-iumchloride
1-acetyl-3-(2,6-dimethyl-phenylamino)-2-methyl-6-nitro-imidazo[1,2-a]pyridin-1-iumchloride
1-acetyl-5-amino-7-chloro-3-(2,6-dimethyl-phenylamino)-2-thiophene-2-yl-imidazo[1,2-a]pyrimidin-1-iumchloride
1-acetyl-3-cyclohexylamino-2-furan-2-yl-imidazo[1,2-a]pyridin-1-iumchloride hydrochloride.

14. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of pain.

15. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of drug and/or alcohol abuse.

16. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of diarrhoea.

17. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of gastritis.

18. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of ulcers.

19. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of urinary incontinence.

20. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of depression.

21. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of narcolepsy.

22. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of excess weight.

23. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of asthma.

24. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of glaucoma.

25. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of tinnitus.

26. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of itching.

27. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment of the hyperkinetic syndrome.

28. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment or prophylaxis of epilepsy.

29. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for the treatment or prophylaxis of schizophrenia.

30. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition for anxiolysis.

31. Use of salts of a bicyclic, N-acylated imidazo-3-amine and imidazo-5-amine of general Formula I according to claim 1 and/or their addition products with physiologically acceptable acids according to claim 6 in which R¹ to R⁸ A and X have the meaning given in claim 1 for producing a pharmaceutical composition with anaesthetic activity.

## Revendications

1. Sels d'imidazo-3-amines et imidazo-5-amines N-acylées bicycliques de formule générale I, 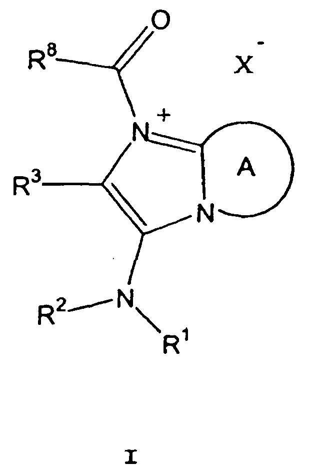 dans laquelle
R¹ représente un reste tertiobutyle, 1,1,3,3-tétraméthylbutyle, (CH₂)₆CN, cyclohexyle, CH₂CO-(O-méthyle), 2,6-diméthylphényle, tertiobutyle ou CH₂CH₂R (R = 4-morpholino),
R² représente l'hydrogène, un groupe COR^{b} dans lequel R^{b} représente un reste alkyle en C₁ à C₈ linéaire ou ramifié ou un reste cycloalkyle en C₃ à C₈,
R³ est choisi dans le groupe des restes 4-acétamidophényle, 2-bromophényle, 3-bromophényle, 4-bromophényle, 4-bromo-2-fluorophényle, 5-bromo-2-fluorophényle, 3-bromo-4-fluorophényle, 4-tertiobutylphényle, 2-chloro-4-fluorophényle, 2-chloro-6-fluorophényle, 2-chlorophényle, 3-chlorophényle, 4-chlorophényle, 4-cyanophényle, 2,3-dichlorophényle, 2,4-dichlorophényle, 3,4-dichlorophényle, 2,3-diméthoxyphényle, 3,4-diméthoxyphényle, 2,4-diméthylphényle, 2,5-diméthylphényle, 2-fluorophényle, 3-fluorophényle, 4-fluorophényle, 4-hexylphényle, 3-hydroxyphényle, 2-méthoxyphényle, 2-méthylphényle, 3-méthylphényle, 4-méthylphényle, 4-nitrophényle, 3-phénoxyphényle, 4-(1-pyrrolidino)-phényle, 2-(trifluorométhyl)-phényle, 3-(trifluorométhyl)-phényle, 4-(trifluorométhyl)-phényle, 3,4,5-triméthoxyphényle, 3-(4-chlorophénoxy)-phényle, 4-acétoxy-3-méthoxyphényle, 4-diméthylaminonaphtyle, 2-éthoxynaphtyle, 4-méthoxynaphtyle, 2-(1-(phénylsulfonyl)-pyrrole), 2-(N-méthylpyrrole), 2-(N-(3,5-dichlorophényl)-pyrrole, 2-(1-(4-chlorophényl)-pyrrole), 2-(5-acétoxyméthylfurfuryle), 2-(5-méthylfurfuryle), 2-(5-nitrofurfuryle), 2-[5-(3-nitrophényl)-furfuryle], 2-[5-(2-nitrophényl)-furfuryle], 2-(5-bromofurfuryle), 2-[5-(4-chlorophényl)-furfuryle], 2-(4,5-diméthylfurfuryle), 2-[5-(2-chlorophényl)-furfuryle],2-(5-éthylfurfuryle)-, 2-[5-(1,3-dioxalanefurfuryle], 2-(5-chlorothiophényle)-, 2-(5-méthylthiophényle), 2-(5-éthylthiophényle), 2-(3-méthylthiophényle), 2-(4-bromothiophényle), 2-(5-nitrothiophényle), 5-(2-carboxythiophényle), 2-[4-(phényléthyl)-thiophényle], 2-[5-(méthylthio)-phényle], 2-(3-bromothiophényle), 2-(3-phénoxythiophényle) ou 2-(5(bromothiophényle) ou en particulier méthyle, cyclohexyle, phényle, furanne-2-yle, 2-pyridyle ou 2-thiophényle,
A est un fragment de noyau à trois chaînons choisi pour représenter l'une des formules 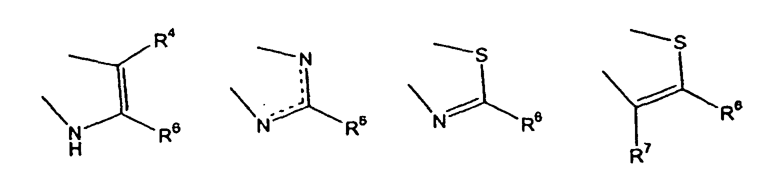 ou un fragment de noyau à quatre chaînons choisi pour représenter l'une des formules 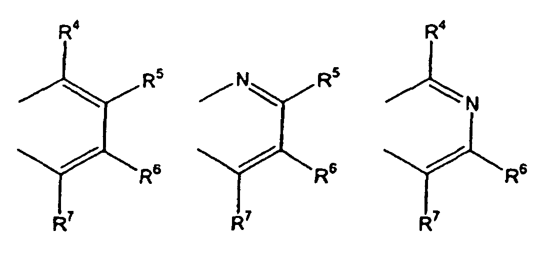 où R⁴, R⁵, R⁶ et R⁷ représentent dans chaque cas, indépendamment les uns des autres, l'hydrogène, un reste alkyle en C₁ à C₈ linéaire ou ramifié, le fluor, le chlore, le brome, un reste CF₃, CN, NO₂, NHR^{f}, où R^{f} représente l'hydrogène, un radical alkyle en C₁ à C₈ linéaire ou ramifié ou un radical COR''' (R''' = radical alkyle en C₁ à C₄ linéaire ou ramifié ou phényle), un groupe SR⁹, dans lequel R⁹ représente l'hydrogène, un radical alkyle en C₁ à C₄ linéaire ou ramifié, un radical phényle, pyridine, benzyle ou fluorényle, un groupe OR^{h}, dans lequel R^{h} représente l'hydrogène, un radical alkyle en C₁ à C₈ linéaire ou ramifié, un radical COR''' (R''' = alkyle en C₁ à C₄ linéaire ou ramifié ou phényle) ou un radical CO(OR') (R' = alkyle en C₁ à C₈ linéaire ou ramifié), un groupe CO(ORⁱ) ou CH₂CO(ORⁱ), dans lequel Rⁱ désigne dans chaque cas un radical alkyle en C₁ à C₈ linéaire ou ramifié ou un radical phényle éventuellement substitué, ou bien R⁶ et R⁷ forment ensemble un fragment de noyau -CRⁱ=CR^{j}-CH=CH- dans lequel Rⁱ et R^{j} représentent l'hydrogène, ou bien l'un des deux restes Rⁱ et R^{j} est différent de l'hydrogène et représente F, Cl, Br, I ou un radical alkyle en C₁ à C₈ linéaire ou ramifié, tandis que R⁴ et R⁵ ont indépendamment l'un de l'autre la définition indiquée ci-dessus,
R⁸ représente un reste alkyle en C₁ à C₈ linéaire ou ramifié, aryle, hétéroaryle, cycloalkyle en C₃ à C₈ ou un reste aryle, hétéroaryle ou cycloalkyle en C₃ à C₈ lié par l'intermédiaire d'un radical alkylène en C₁ à C₃, et
X désigne l'anion d'un acide inorganique ou organique.

2. Sels d'imidazo-3-amines et imidazo-5-amines N-acylées bicycliques suivant la revendication 1, **caractérisés en ce que** A est un fragment de noyau à trois chaînons de formule 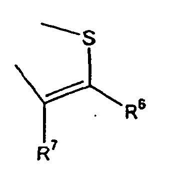 ou un fragment de noyau à quatre chaînons choisi pour représenter l'une des formules

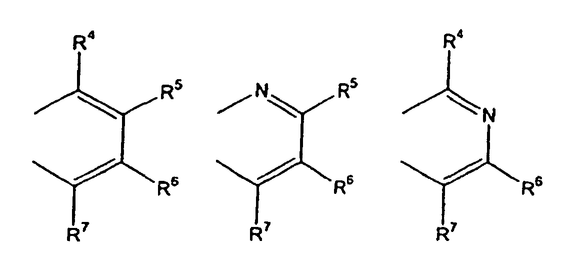

3. Sels d'imidazo-3-amines et imidazo-5-amines N-acylées bicycliques suivant la revendication 1 ou 2, **caractérisés en ce que** R⁴, R⁵, R⁶ et R⁷ sont choisis, indépendamment les uns des autres, dans le groupe comprenant l'hydrogène et les restes méthyle, éthyle, isopropyle, n-propyle, n-butyle, le fluor, le brome, les restes CF₃, CN, NO₂, NHR^{f}, où R^{f} représente l'hydrogène, un radical méthyle, éthyle, n-propyle, n-butyle, C(O)méthyle ou C(O)phényle, le reste SR^{g}, où R^{g} représente l'hydrogène, un radical méthyle, éthyle, n-propyle, n-butyle ou phényle, le reste OR^{h}, où R^{h} représente l'hydrogène, un radical méthyle, éthyle, n-propyle, n-butyle, phényle, CO-méthyle, CO-phényle, CO-(O-méthyle) ou CO-(O-éthyle), le reste CO(ORⁱ) ou CH₂CO(ORⁱ), où Rⁱ désigne dans chaque cas un radical méthyle, éthyle, n-propyle, n-butyle ou phényle, l'hydrogène, le reste méthyle, le chlore, le reste NH₂ et le reste NO₂ étant particulièrement appréciés.

4. Sels d'imidazo-3-amines et imidazo-5-amines N-acylées bicycliques suivant la revendication 1, 2 ou 3, **caractérisés en ce que** R⁸ est un reste méthyle, éthyle, n-propyle, n-butyle, n-pentyle, n-hexyle, un reste phényle non substitué, monosubstitué en position 4 ou disubstitué en positions 2 et 6, un reste cyclohexyle ou un reste 2-naphtyle, les restes méthyle, n-hexyle, 2,6-dichlorophényle, 4-méthoxyphényle, cyclohexyle ou 2-naphtyle étant particulièrement appréciés.

5. Sels d'imidazo-3-amines et imidazo-5-amines N-acylées bicycliques suivant l'une des revendications 1, 2, 3 et 4, **caractérisés en ce que** X représente l'anion de l'acide bromhydrique, de l'acide sulfurique, de l'acide méthanesulfonique, de l'acide formique, de l'acide acétique, de l'acide oxalique, de l'acide succinique, de l'acide tartrique, de l'acide mandélique, de l'acide fumarique, de l'acide lactique, de l'acide citrique, de l'acide glutamique ou de l'acide aspartique ou en particulier de l'acide chlorhydrique.

6. Sels d'imidazo-3-amines et imidazo-5-amines N-acylées bicycliques suivant l'une des revendications 1, 2, 3, 4 ou 5 sous forme de leurs produits d'addition avec des acides, avantageusement avec des acides acceptables du point de vue physiologique.

7. Sels d'imidazo-3-amines et imidazo-5-amines N-acylées bicycliques suivant l'une des revendications 1, 2, 3, 4 ou 5 ou leurs produits d'addition avec des acides suivant la revendication 6, **caractérisés en ce qu'**ils sont choisis dans le groupe suivant :
chlorure de 1-acétyl-3-tertiobutylamino-2-phénylimidazo-[1,2-a]pyridine-1-ium
chlorure de 1-acétyl-3-tertiobutylamino-2-phénylimidazo-[1,2-a]pyrazine-1-ium
chlorure de 1-acétyl-3-tertiobutylamino-2-phénylimidazo-[1,2-a]pyrimidine-1-ium
chlorure de 7-acétyl-5-tertiobutylamino-6-phénylimidazo-[2,1-b]thiazole-7-ium
chlorure de 3-acétyl-1-cyclohexylamino-2-phénylimidazo-[1,2-a]quinoléine-3-ium
chlorure de 1-acétyl-3-cyclohexylamino-2-phénylimidazo-[1,2-a]pyrimidine-1-ium
chlorure de 1-acétyl-8-benzyloxy-3-cyclohexylamino-2-méthylimidazo[1,2-a]pyridine-1-ium
chlorure de 1-acétyl-8-benzyloxy-3-tertiobutylamino-2-méthylimidazo[1,2-a]pyridine-1-ium
chlorure de 1-acétyl-8-benzyloxy-2-méthyl-3-(1,1,3,3-tétraméthylbutylamino)-imidazo[1,2-a]pyridine-1-ium
chlorure de 3-(tertiobutyl-cyclohexanecarbonylamino)-1-cyclohexanecarbonyl-2-phénylimidazo[1,2-a]pyridine-1-ium
chlorure de 3-(tertiobutyl-heptanoylamino)-1-heptanoyl-2-phénylimidazo[1,2-a]pyridine-1-ium
chlorure de 1-acétyl-3-cyclohexylamino-2-furanne-2-yl-imidazo[1,2-a]pyridine-1-ium
chlorure de 1-acétyl-3-cyclohexylamino-2-furanne-2-yl-imidazo[1,2-a]pyrimidine-1-ium
chlorure de 1-acétyl-5-amino-7-chloro-3-cyclohexylamino-2-furanne-2-ylimidazo[1,2-a]pyrimidine-1-ium
chlorure de 1-acétyl-3-cyclohexylamino-2-pyridine-2-yl-imidazo[1,2-a]pyridine-1-ium
chlorure de 1-acétyl-3-cyclohexylamino-6-nitro-2-pyridine-2-ylimidazo[1,2-a]pyridine-1-ium
chlorure de 1-acétyl-3-cyclohexylamino-5-méthyl-2-pyridine-2-ylimidazo[1,2-a]pyridine-1-ium
chlorure de 1-acétyl-3-cyclohexylamino-2-pyridine-2-yl-imidazo[1,2-a]pyrazine-1-ium
chlorure de 1-acétyl-3-cyclohexylamino-2-pyridine-2-yl-imidazo[1,2-a]pyrimidine-1-ium
chlorure de 1-acétyl-5-amino-7-chloro-3-cyclohexylamino-2-pyridine-2-ylimidazo[1,2-a]pyrimidine-1-ium
chlorure de 1-acétyl-3-cyclohexylamino-5,7-diméthyl-2-pyridine-2-ylimidazo[1,2-a]pyrimidine-1-ium
chlorure de 7-acétyl-5-cyclohexylamino-6-pyridine-2-yl-imidazo[2,1-b]thiazole-7-ium
chlorure de 1-acétyl-2-cyclohexyl-3-cyclohexylamino-imidazo[1,2-a]pyridine-1-ium
chlorure de 1-acétyl-3-cyclohexylamino-2-méthylimidazo-[1,2-a]pyrimidine-1-ium
chlorure de 1-acétyl-5-amino-7-chloro-3-cyclohexylamino-2-méthylimidazo[1,2-a]pyrimidine-1-ium
chlorure de 1-acétyl-5-amino-7-chloro-3-cyclohexylamino-2-thiophène-2-ylimidazo[1,2-a]pyrimidine-1-ium
chlorure de 1-acétyl-5-amino-7-chloro-2-furanne-2-yl-3-(méthoxycarbonylméthylamino)-imidazo[1,2-a)pyrimidine-1-ium
chlorure de 1-acétyl-2-cyclohexyl-3-(méthoxycarbonylméthylamino)-imidazo[1,2-a]pyridine-1-ium
chlorure de 1-acétyl-2-cyclohexyl-3-(méthoxycarbonylméthylamino)-imidazo[1,2-a]pyrimidine-1-ium
chlorure de 1-acétyl-3-(2,6-diméthylphénylamino)-2-méthyl-6-nitro-imidazo[1,2-a]pyridine-1-ium
chlorure de 1-acétyl-5-amino-7-chloro-3-(2,6-diméthylphénylamino)-2-thiophène-2-ylimidazo[1,2-a]pyrimidine-1-ium chlorure de 1-acétyl-3-cyclohexylamino-2-furanne-2-ylimidazo[1,2-a]pyridine-1-ium, chlorhydrate.

8. Procédé de production des sels d'imidazo-3-amines et imidazo-5-amines N-acylées bicycliques et de leurs produits d'addition d'acides suivant l'une des revendications 1 à 7, comprenant les étapes suivantes
a) préparation des imidazo-3-amines et imidazo-5-amines par une réaction entre trois composants formés d'amidine, d'aldéhyde et d'isonitrile dans un solvant, avantageusement le dichlorométhane, et en présence d'un acide, avantageusement l'acide perchlorique, les composés de départ étant ajoutés successivement dans l'ordre amidine, aldéhyde et isonitrile,
b) le cas échéant, pour la préparation de composés dans lesquels R² ne représente pas l'hydrogène, réaction des produits formés dans l'étape a) avec un composé R²Hal (Hal = brome, iode ou en particulier chlore) ou avec un isocyanate,
c) réaction du produit réactionnel de l'étape a) ou b) avec un excès avantageusement au moins quatre fois molaire d'un chlorure d'acide R⁸C(O)Cl,
d) après la fin de la réaction, élimination de l'excès de chlorure d'acide du mélange réactionnel,
e) le cas échéant, échange d'une manière connue du chlorure contre un autre reste d'acide, avantageusement d'acide bromhydrique, d'acide sulfurique, d'acide méthanesulfonique, d'acide formique, d'acide acétique, d'acide oxalique, d'acide succinique, d'acide tartrique, d'acide mandélique, d'acide fumarique, d'acide lactique, d'acide citrique, d'acide glutamique ou d'acide aspartique,
f) le cas échéant, préparation d'un produit d'addition d'acide, avantageusement d'un acide acceptable du point de vue physiologique.

9. Procédé suivant la revendication 8, **caractérisé en ce que** l'étape c) est conduite dans un solvant formé d'un éther ou d'un hydrocarbure halogéné, avantageusement de THF ou de DCM, à une température de 0 à 60°C.

10. Procédé suivant la revendication 8 ou 9, **caractérisé en ce que** l'étape d) est conduite pendant 2 à 12 heures après addition du chlorure d'acide dans l'étape c).

11. Procédé suivant la revendication 8 ou 9, **caractérisé en ce que** l'élimination de l'excès de chlorure d'acide dans l'étape d) est conduite au moyen d'un accepteur formé d'une résine, en particulier au moyen de (tris-(2-aminoéthyl)-amine)-polystyrène.

12. Médicament contenant comme substance active au moins un sel d'une imidazo-3-amine ou imidazo-5-amine N-acylée bicyclique de formule générale (I) suivant la revendication 1 et/ou au moins un produit d'addition formé avec un acide acceptable du point de vue physiologique suivant la revendication 6, où R¹ à R⁸, A et X ont la définition indiquée dans la revendication 1.

13. Médicament suivant la revendication 12,
**caractérisé en ce qu'**il contient comme substance active au moins un sel d'une imidazo-3-amine ou imidazo-5-amine N-acylée bicyclique ou son produit d'addition d'acide, choisi dans le groupe suivant :
chlorure de 1-acétyl-3-tertiobutylamino-2-phénylimidazo-[1,2-a]pyridine-1-ium
chlorure de 1-acétyl-3-tertiobutylamino-2-phénylimidazo-[1,2-a]pyrazine-1-ium
chlorure de 1-acétyl-3-tertiobutylamino-2-phénylimidazo-[1,2-a]pyrimidine-1-ium
chlorure de 7-acétyl-5-tertiobutylamino-6-phénylimidazo-[2,1-b]thiazole-7-ium
chlorure de 3-acétyl-1-cyclohexylamino-2-phénylimidazo-[1,2-a]quinoléine-3-ium
chlorure de 1-acétyl-3-cyclohexylamino-2-phénylimidazo-[1,2-a]pyrimidine-1-ium
chlorure de 1-acétyl-8-benzyloxy-3-cyclohexylamino-2-méthylimidazo[1,2-a]pyridine-1-ium
chlorure de 1-acétyl-8-benzyloxy-3-tertiobutylamino-2-méthylimidazo[1,2-a]pyridine-1-ium
chlorure de 1-acétyl-8-benzyloxy-2-méthyl-3-(1,1,3,3-tétraméthylbutylamino)-imidazo[1,2-a]pyridine-1-ium
chlorure de 3-(tertiobutyl-cyclohexanecarbonylamino)-1-cyclohexanecarbonyl-2-phénylimidazo[1,2-a]pyridine-1-ium
chlorure de 3-(tertiobutyl-heptanoylamino)-1-heptanoyl-2-phénylimidazo[1,2-a]pyridine-1-ium
chlorure de 1-acétyl-3-cyclohexylamino-2-furanne-2-yl-imidazo[1,2-a]pyridine-1-ium
chlorure de 1-acétyl-3-cyclohexylamino-2-furanne-2-yl-imidazo[1,2-a]pyrimidine-1-ium
chlorure de 1-acétyl-5-amino-7-chloro-3-cyclohexylamino-2-furanne-2-ylimidazo[1,2-a]pyrimidine-1-ium
chlorure de 1-acétyl-3-cyclohexylamino-2-pyridine-2-yl-imidazo[1,2-a]pyridine-1-ium
chlorure de 1-acétyl-3-cyclohexylamino-6-nitro-2-pyridine-2-ylimidazo[1,2-a]pyridine-1-ium
chlorure de 1-acétyl-3-cyclohexylamino-5-méthyl-2-pyridine-2-ylimidazo[1,2-a]pyridine-1-ium
chlorure de 1-acétyl-3-cyclohexylamino-2-pyridine-2-yl-imidazo[1,2-a]pyrazine-1-ium
chlorure de 1-acétyl-3-cyclohexylamino-2-pyridine-2-yl-imidazo[1,2-a]pyrimidine-1-ium
chlorure de 1-acétyl-5-amino-7-chloro-3-cyclohexylamino-2-pyridine-2-ylimidazo[1,2-a]pyrimidine-1-ium
chlorure de 1-acétyl-3-cyclohexylamino-5,7-diméthyl-2-pyridine-2-ylimidazo[1,2-a]pyrimidine-1-ium
chlorure de 7-acétyl-5-cyclohexylamino-6-pyridine-2-yl-imidazo[2,1-b]thiazole-7-ium
chlorure de 1-acétyl-2-cyclohexyl-3-cyclohexylamino-imidazo[1,2-a]pyridine-1-ium
chlorure de 1-acétyl-3-cyclohexylamino-2-méthylimidazo[1,2-a]pyrimidine-1-ium
chlorure de 1-acétyl-5-amino-7-chloro-3-cyclohexylamino-2-méthylimidazo[1,2-a]pyrimidine-1-ium
chlorure de 1-acétyl-5-amino-7-chloro-3-cyclohexylamino-2-thiophène-2-ylimidazo[1,2-a]pyrimidine-1-ium
chlorure de 1-acétyl-5-amino-7-chloro-2-furanne-2-yl-3-(méthoxycarbonylméthylamino)-imidazo[1,2-a]pyrimidine-1-ium
chlorure de 1-acétyl-2-cyclohexyl-3-(méthoxycarbonylméthylamino)-imidazo[1,2-a]pyridine-1-ium
chlorure de 1-acétyl-2-cyclohexyl-3-(méthoxycarbonylméthylamino)-imidazo[1,2-a]pyrimidine-1-ium
chlorure de 1-acétyl-3-(2,6-diméthylphénylamino)-2-méthyl-6-nitro-imidazo[1,2-a]pyridine-1-ium
chlorure de 1-acétyl-5-amino-7-chloro-3-(2,6-diméthylphénylamino)-2-thiophène-2-ylimidazo[1,2-a]pyrimidine-1-ium chlorure de 1-acétyl-3-cyclohexylamino-2-furanne-2-ylimidazo[1,2-a]pyridine-1-ium, chlorhydrate.

14. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de la douleur.

15. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement d'un abus de drogue et/ou d'alcool.

16. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de la diarrhée.

17. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de la gastrite.

18. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement d'ulcères.

19. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de l'incontinence urinaire.

20. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de dépressions.

21. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de la narcolepsie.

22. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de la surcharge pondérale.

23. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement de l'asthme.

24. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement du glaucome.

25. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement des acouphènes.

26. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement du prurit.

27. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement du syndrome hypercinétique.

28. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement ou à la prophylaxie de l'épilepsie.

29. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné au traitement ou à la prophylaxie de la schizophrénie.

30. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament destiné à l'anxiolyse.

31. Utilisation de sels d'une imidazo-3-amine ou imidazo-5-amine N-acylée bicyclique de formule générale I suivant la revendication 1 et/ou de leurs produits d'addition d'acides acceptables du point de vue physiologique suivant la revendication 6, dans lesquels R¹ à R⁸, A et X ont la définition indiquée dans la revendication 1, pour la préparation d'un médicament à effet anesthésique.
